(19) **Europäisches Patentamt**
**European Patent Office**
**Office européen des brevets**

(11) **EP 3 951 789 B1**

(12) **EUROPEAN PATENT SPECIFICATION**

(45) Date of publication and mention
of the grant of the patent:
**24.12.2025 Bulletin 2025/52**

(21) Application number: **21189767.3**

(22) Date of filing: **05.08.2021**

(51) International Patent Classification (IPC):
**G16H 20/40** (2018.01)     **G16H 40/63** (2018.01)
**G16H 50/20** (2018.01)     **G16H 70/20** (2018.01)

(52) Cooperative Patent Classification (CPC):
**G16H 50/20; G06N 3/0442; G06N 3/0455;
G06N 3/0464; G06N 3/0495; G06N 3/09;
G16H 20/40; G16H 40/63; G16H 70/20**

(54) **USE OF MACHINE LEARNING TO IMPROVE WORKFLOW DURING MAPPING AND TREATMENT OF CARDIAC ARRHYTHMIAS**

VERWENDUNG VON MASCHINENLERNEN ZUR VERBESSERUNG DES ARBEITSFLUSSES WÄHREND DER ABBILDUNG UND BEHANDLUNG VON HERZARRHYTHMIEN

UTILISATION DE L'APPRENTISSAGE MACHINE POUR AMÉLIORER LE FLUX DE TRAVAIL PENDANT LE MAPPAGE ET LE TRAITEMENT D'ARYTHMIES CARDIAQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.08.2020 US 202063062063 P
27.07.2021 US 202117443774**

(43) Date of publication of application:
**09.02.2022 Bulletin 2022/06**

(73) Proprietor: **Biosense Webster (Israel) Ltd.
Yokneam, 2066717 (IL)**

(72) Inventors:
• **BOTZER, Lior**
  **2066717 Yokneam (IL)**
• **RAVUNA, Eliyahu**
  **2066717 Yokneam (IL)**
• **YARNITSKY, Jonathan**
  **2066717 Yokneam (IL)**

• **NAKAR, Elad**
  **2066717 Yokneam (IL)**
• **GOLDBERG, Stanislav**
  **2066717 Yokneam (IL)**
• **AMIT, Matityahu**
  **2066717 Yokneam (IL)**
• **AMOS, Yariv Avraham**
  **2066717 Yokneam (IL)**
• **ELIYAHU, Shiran**
  **2066717 Yokneam (IL)**
• **AUERBACH, Shmuel**
  **2066717 Yokneam (IL)**
• **KAUFMAN, Ana**
  **2066717 Yokneam (IL)**

(74) Representative: **Carpmaels & Ransford LLP
One Southampton Row
London WC1B 5HA (GB)**

(56) References cited:
**EP-A2- 2 945 087**     **WO-A1-2018/134143**
**US-A1- 2011 201 900**     **US-A1- 2013 132 117**

# EP 3 951 789 B1

**Description**

BACKGROUND

Conventional systems for cardiac mapping and ablation include many different options and capabilities, the majority of which are not used during mapping and treatment of cardiac arrhythmias. For example, conventional cardiac mapping and ablation systems provide a variety of different visualization methods, where each physician and/or clinical account specialist ("CAS") has preferred visualizations with respect to certain graphical user interfaces ("GUIs") or pop-up windows to use during cardiac arrhythmia treatment. For instance, a GUI of a conventional cardiac mapping and ablation system can be very busy and complex, in that the GUI presents many different options to each physician and/or CAS including different screens, pop-up windows, visualizations, templates, presentations, menus, and tools. Often, these numerous options are not presented or organized in a manner that is intuitive to the physician and/or CAS and easy for the physician and/or CAS to locate. Many of these options may never be used by a particular physician and/or CAS during a mapping and treatment of cardiac arrhythmias due to personal preferences or for other reasons.

Further, each physician and/or CAS has preferences or style as to how data is visualized and which cardiac map to use during different phases of a cardiac arrhythmia treatment. For example, each physician and/or CAS may have preferences regarding which pop-up windows are to be retained on a screen and/or a manner in which data is visualized. In addition, each patient has his or her unique arrhythmia and other unique health demographics. Prior to, or even during a cardiac arrhythmia treatment, the physician and/or CAS may spend considerable time locating his or her preferred options based upon the mapping and treatment to be performed, a patient's unique health demographics, and a preferred approach to conducting the procedure.

Yet, conventional cardiac mapping and ablation systems are unable to be customized to provide the physicians and CASs with GUIs that are specific to their needs and intuitive.

US 2011/201900 A1 describes a patient treatment monitoring system that includes an interface for receiving multiple different types of patient medical information including data derived from a patient monitoring device and a patient medical imaging device. A data processor processes the received multiple different types of patient medical information to be suitable for presentation in a display image. A display processor initiates generation of data representing a single composite display image including an image element representing multiple sequentially performed individual stages of a treatment process. The individual stages are associated with corresponding different sets of the received multiple different types of patient medical information. The single composite display image includes multiple image areas for displaying one of the corresponding different sets of the received multiple different types of patient medical information, in response to user selection of a particular stage of the individual stages using the image element.

WO 2018/134143 A1 describes an augmented reality system that includes a head-mountable augmented reality platform having a display to provide guidance to a user. A prediction module is trained in a procedure and is configured to predict a next activity in the procedure based on a current condition. An image generation module is responsive to the prediction module to provide a visual cue of a predicted next activity to the user through the head-mountable augmented reality platform.

SUMMARY

The invention is defined by the system of claim 1 and the method of claim 4. Optional aspects of the invention are presented in the dependent claims.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 illustrates a diagram of an exemplary system in which one or more features of the disclosure subject matter can be implemented according to one or more embodiments;

FIG. 2 illustrates a block diagram of an example system for monitoring and communicating patient biometrics according to one or more embodiments;

FIG. 3 illustrates a block diagram of a method according to one or more embodiments;

FIG. 4 illustrates a graphical depiction of an artificial intelligence system according to one or more embodiments;

FIG. 5 illustrates an example of a neural network and a block diagram of a method performed in the neural network according to one or more embodiments;

FIG. 6 illustrates a diagram of an environment in which an exemplary system is utilized according to one or more embodiments;

FIG. 7 illustrates a block diagram of a method according to one or more embodiments; and

FIG. 8 illustrates a structure of a recurrent neural network (RNN) according to one or more embodiments.

DETAILED DESCRIPTION

Disclosed herein is a machine learning and artificial intelligence (ML/AI) method and system. More particularly, the present teachings relate to a ML/AI algorithm that improves workflows during mapping and treating patients.

The ML/AI method and system can be a processor executable code or software that is necessarily rooted in process operations by, and in processing hardware of, medical device equipment to generate and present image displays, during mapping and treating of cardiac conditions, to one or more users on a graphic user interface. Examples of medical device equipment include, but are not limited to, a cardiac electrophysiology system, a cardiac mapping system, a cardiac arrhythmia treatment system, an ablation system, a cardiac mapping and ablation system, similar systems, and combination thereof.

According to one or more embodiments, a mapping engine is an implementation of the ML/AI method and system described herein. The mapping engine can be part of medical device equipment and can provide a specific multi-step data manipulation of biometric data, user preferences, and health demographics for automatic patient mapping and condition treating (e.g., ablating of cardiac tissue), as well as improve workflows by customizing the patient mapping and the condition treating. For example, during the patient mapping and the condition treating, the mapping engine generally presents personalized graphical user interfaces and intelligent toolbars as part of the medical device equipment that are custom tailored based upon the biometric data, the user preferences, and the health demographics.

For example, the mapping engine can receive and monitor historical data regarding medical procedure workflow preferences previously exhibited by one or more users conducting cardiac electrophysiology procedures on a plurality of patients; receive and monitor unique physiological characteristics of arrhythmias presented by the plurality of patients undergoing the cardiac electrophysiology procedures; receive and monitor personal health demographics of the plurality of patients; implement a machine learning (ML) model to learn the medical procedure workflow preferences, the unique physiological characteristics, and the personal health demographics of the plurality of patients; and present image display options to the one or more users during present cardiac electrophysiology procedures.

According to one or more advantages, technical effects, and/or benefits, the mapping engine can implement ML and data compression of the biometric data, the user preferences, and the health demographics, as well as the maps, visualizations, and interfaces, to reduce resources needed for storage and sharing of electrocardiograph information obtained during mapping and treatment of cardiac conditions. Further, according to one or more advantages, technical effects, and/or benefits, the mapping engine can present screens, templates, functions, and tools in a GUI to a physician in a more educated manner that is personalized to that physician's preferences and the unique characteristics of a current patient's arrhythmia and personal health demographics. Furthermore, the mapping engine can adjust the GUI based on relevant arrhythmia type, relevant physician's preferences, and patient's demographics. In this manner, the mapping engine improves workflow efficiency resulting in mapping and ablation procedures that are smoother, faster, and more successful. More particularly, through ML by the mapping engine, behaviors and preferences of different physician are identified and selected based upon prior mapping and ablation procedures conducted by those different physicians. The mapping engine arranges image display options in the GUI based upon the physician's past behavior and preferences, thus reducing the time needed for the physician to locate these particular options during the mapping and ablation steps.

According to one or more advantages, technical effects, and/or benefits, once the mapping engine determined that a particular workflow smoother and more efficient that another workflow, the mapping engine can teach or suggest the workflow to other physicians who may be struggling with treatments involving certain types of arrhythmias or other challenges. Further, the mapping engine can adjust the GUI during the workflow or procedure by opening and closing windows, settings, transparency, changing maps, etc. Based upon the activities and signals that are generated during the course of the cardiac ablation, the mapping engine can suggest to the physician what type of an arrhythmia is occurring within the patient. In response, the physician can either confirm or decline the accuracy of the diagnosis. In the event the physician confirms that the mapping engine is correct, then the mapping engine may utilize its previous learnings obtained from prior procedures conducted by this physician or other physicians to suggest the use of different tools as the workflow continues. For example, if the physician is mapping a flutter type of arrhythmia, which has been diagnosed by the mapping engine and confirmed by the physician, the mapping engine may ask the physician whether they wants to open a tool or feature specifically intended for treating that particular flutter type. The mapping engine may then suggest to the physician different settings for confidence mapping.

According to one or more advantages, technical effects, and/or benefits, the mapping engine can improve medical procedures, such as a pulmonary vein (PV) isolation procedure (e.g., a type of cardiac ablation procedure that is intended to stop electrical signals in the heart that are causing heart rhythm problems). During the course of the PV isolation procedure, the physician may follow a 'recipe' in step-by-step fashion or deviate from therefrom based upon personal style or in the event an unexpected circumstance arises during the PV isolation procedure. Through ML, the mapping engine can recognize steps that are common to the PV isolation procedure performed by most physicians, as well as recognize steps where physicians deviate from the common procedure steps or are completely unrelated. As described herein, using this information, the mapping engine can learn a particular physician's style and suggest next steps in the PV isolation procedure that match that physician's particular style. Also, using this information, the mapping engine may suggest to the physician an approach that differs from the physician's style, however has been shown to be successful with other physicians in the past. Also, using this information, the mapping engine can compare the different methods of PV isolation performed by different physicians to determine whether one method is superior over another from the standpoint of smoothing workflow or increasing successful patient outcomes. By identifying PV isolation procedures that have had successful outcomes in the past, these procedures may be taught or suggested to other physicians who may be struggling with obtaining successful outcomes. Also, when a physician is isolating the PV, the mapping engine can identify when the physician has completed the ablation line and may suggest a next step in the procedure, such as inserting a lasso catheter or another mapping catheter, both of which steps are not menu specific.

According to one or more embodiments, a system is provided. The system includes a memory configured to store processor executable program instructions of a mapping engine. The system also includes at least one processor configured to execute the program instructions of the mapping engine to cause the system to receive information with respect to initiating a medical procedure for a patient, predict workflow preferences for one or more users performing the medical procedure from the information by employing a model of the mapping engine; and generate, using the workflow preferences, image display options to the one or more users for presentation by the system. The information includes health demographics and biometric data of the patient.

According to one or more embodiments or any of the system embodiments herein, the image display options can be presented to the one or more users via one or more displays during the medical procedure, and at least one of the image display options can include an intelligent toolbar.

According to one or more embodiments or any of the system embodiments herein, the system can include an eye tracking apparatus worn by a first user of the one or more users while conducting the medical procedure. The eye tracking apparatus can monitor and obtain present workflow preferences of the first user.

According to one or more embodiments or any of the system embodiments herein, the at least one processor can be further configured to execute the program instructions of the mapping engine to cause the system to initiate storage of the information relating to the medical procedure to a storage device in advance of a predicted time for completion of the medical procedure.

According to one or more embodiments or any of the system embodiments herein, the biometric data can include unique physiological characteristics of one or more arrhythmias of the patient.

According to one or more embodiments or any of the system embodiments herein, the model of the mapping engine can include a trained deep learning architecture using a recurrent neural network.

According to one or more embodiments or any of the system embodiments herein, the model of the mapping engine can be trained based upon an analysis of a plurality of prior cardiac electrophysiology cases.

According to one or more embodiments or any of the system embodiments herein, the at least one processor can be further configured to execute the program instructions of the mapping engine to cause the system to receive historical data regarding past medical procedures on a plurality of patients, the historical data including workflow preferences of a plurality of users performing the past medical procedures, biometric data of the plurality of patients, and health demographics of the plurality of patients; and generate and train by the model using the historical data

According to one or more embodiments or any of the system embodiments herein, the at least one processor can be further configured to execute the program instructions of the mapping engine to cause the system to predict next events to be performed by the one or more user conducting the medical procedure on the patient.

According to one or more embodiments or any of the system embodiments herein, the at least one processor can be further configured to execute the program instructions of the mapping engine to cause the system to predict probabilities of the next events and generate, within the image display options, input/output elements for the next events based on the probabilities for selection by the user.

According to one or more embodiments, a method is provided. The method includes receiving, by a mapping engine executed by at least one processor, information with respect to initiating a medical procedure for a patient, the information comprising health demographics and biometric data of the patient; predicting, by the mapping engine, workflow preferences for one or more users performing the medical procedure from the information by employing a model of the mapping engine; and generating, by the mapping engine using the workflow preferences, image display options to the one or more users for presentation by the system.

According to one or more embodiments or any of the method embodiments herein, the image display options can be presented to the one or more users via one or more displays during the medical procedure, and the at least one of the image display options can include an intelligent toolbar.

According to one or more embodiments or any of the method embodiments herein, an eye tracking apparatus can monitor and obtain present workflow preferences of a first user of the one or more. The first user can wear the eye tracking apparatus while conducting the medical procedure.

According to one or more embodiments or any of the method embodiments herein, the method can include initiating storage of the information relating to the medical procedure to a storage device in advance of a predicted time for completion of the medical procedure.

According to one or more embodiments or any of the method embodiments herein, the biometric data can include unique physiological characteristics of one or more arrhythmias of the patient.

According to one or more embodiments or any of the method embodiments herein, the model of the mapping engine can include a trained deep learning architecture using a recurrent neural network.

According to one or more embodiments or any of the method embodiments herein, the model of the mapping engine can be trained based upon an analysis of a plurality of prior cardiac electrophysiology cases.

According to one or more embodiments or any of the method embodiments herein, the method can include receiving historical data regarding past medical procedures on a plurality of patients, the historical data including workflow preferences of a plurality of users performing the past medical procedures, biometric data of the plurality of patients, and health demographics of the plurality of patients; and generating and training by the model using the historical data.

According to one or more embodiments or any of the method embodiments herein, the method can include predicting next events to be performed by the one or more user conducting the medical procedure on the patient.

According to one or more embodiments or any of the method embodiments herein, the method can include predicting probabilities of the next events and generating, within the image display options, input/output elements for the next events based on the probabilities for selection by the user.

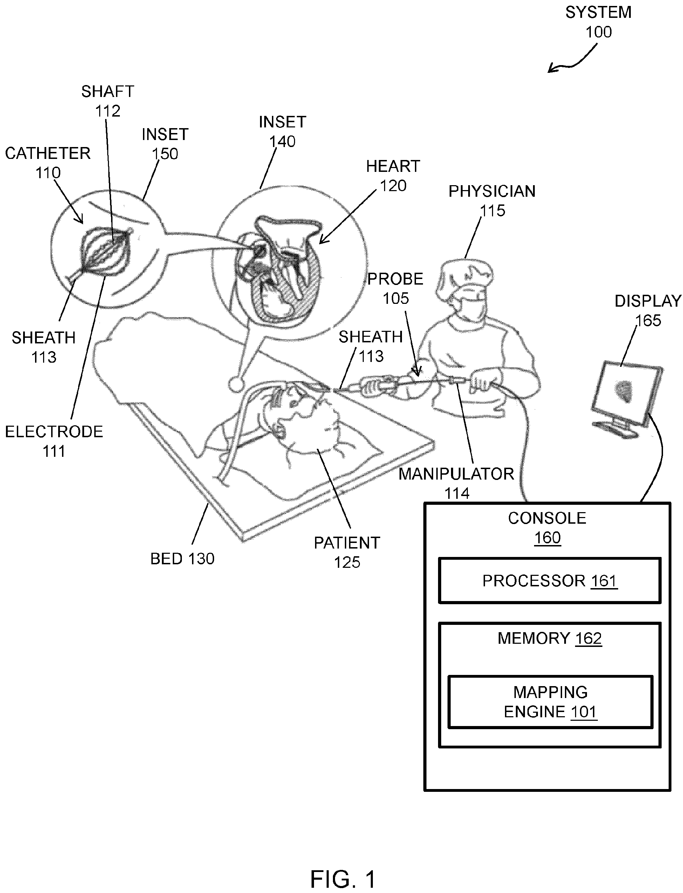

FIG. 1 is a diagram of an exemplary system (e.g., medical device equipment), shown as a system 100, in which one or more features of the subject matter herein can be implemented according to one or more embodiments. The system 100, as an example of medical device equipment, can be a cardiac electrophysiology system, a cardiac mapping system, a cardiac arrhythmia treatment system, an ablation system, a cardiac mapping and ablation system, or combination thereof.

All or part of system 100 can be used to collect information (e.g., biometric data and/or a training dataset) and/or used to implement a mapping engine 101 (e.g., a ML/AI algorithm) as described herein. The mapping engine 101 can be considered a ML/AI algorithm as described herein.

The system 100, as illustrated, includes a probe 105 with a catheter 110 (including at least one electrode 111), a shaft 112, a sheath 113, and a manipulator 114. The system 100, as illustrated, also includes a physician 115 (or a medical professional or clinician, CAS, or a user in general), a heart 120, a patient 125, and a bed 130 (or a table). Note that insets 140 and 150 show the heart 120 and the catheter 110 in greater detail. The system 100 also, as illustrated, includes a console 160 (including one or more processors 161 and memories 162) and a display 165. Note further each element and/or item of the system 100 is representative of one or more of that element and/or that item. The example of the system 100 shown in FIG. 1 can be modified to implement the embodiments disclosed herein. The disclosed embodiments can similarly be applied using other system components and settings. Additionally, the system 100 can include additional components, such as elements for sensing electrical activity, wired or wireless connectors, processing and display devices, or the like. While the heart 120 is utilized as a primary example, the heart 120 is representative, as any body part, anatomical structure, and/or intra-body organ can be examined, mapped, and/or diagnosed by the system 100. Thus, the probe 105 having shafts (e.g., the shaft 112) that may be navigated by the physician 115 into a body part or intra-body organ, such as the heart 120, of the patient 125 lying on the bed 130. According to embodiments, multiple probes may be provided; however, for purposes of conciseness, a single probe 105 is described herein. Yet, it is understood that the probe 105 may represent multiple probes.

The system 100 can be utilized to detect, diagnose, and/or treat cardiac conditions (e.g., using the mapping engine 101). Cardiac conditions, such as cardiac arrhythmias, persist as common and dangerous medical ailments, especially in the aging population. For instance, the system 100 can be part of a surgical system (e.g., CARTO® system sold by Biosense Webster) that is configured to obtain biometric data (e.g., anatomical and electrical measurements of a patient's organ, such as the heart 120) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 120. Such locating may be done via an electrophysiological investigation during which electrical potentials are detected spatially resolved with a mapping catheter (e.g., the catheter 110) introduced into the chamber of the heart 120. This electrophysiological investigation, the so-called electro-anatomical mapping, thus provides 3D mapping data which can be displayed on the display 165. In many cases, the mapping function and a treatment function (e.g., ablation) are provided by a single catheter or group of catheters such that the mapping catheter also operates as a treatment (e.g., ablation) catheter at the same time. In this case, the mapping engine 101 can be directly stored and executed by the catheter 110.

In patients (e.g., the patient 125) with normal sinus rhythm (NSR), the heart (e.g., the heart 120), which includes atrial, ventricular, and excitatory conduction tissue, is electrically excited to beat in a synchronous, patterned fashion. Note that this electrical excitement can be detected as intracardiac electrocardiogram (IC ECG) data or the like.

In patients (e.g., the patient 125) with a cardiac arrhythmia (e.g., atrial fibrillation or aFib), abnormal regions of cardiac tissue do not follow a synchronous beating cycle associated with normally conductive tissue, which is in contrast to patients with NSR. Instead, the abnormal regions of cardiac tissue aberrantly conduct to adjacent tissue, thereby disrupting the cardiac cycle into an asynchronous cardiac rhythm. Note that this asynchronous cardiac rhythm can also be detected as the IC ECG data. Such abnormal conduction has been previously known to occur at various regions of the heart 120, for example, in the region of the sino-atrial (SA) node, along the conduction pathways of the atrioventricular (AV) node, or in the cardiac muscle tissue forming the walls of the ventricular and atrial cardiac chambers. There are other conditions, such as flutter, where the pattern of abnormally conducting tissues lead to reentry paths such that the chamber beats in a regular pattern that can be multiple times the sinus rhythm.

In support of the system 100 detecting, diagnosing, and/or treating cardiac conditions, the probe 105 can be navigated by the physician 115 into the heart 120 of the patient 125 lying on the bed 130. For instance, the physician 115 can insert the shaft 112 through the sheath 113, while manipulating a distal end of the shaft 112 using the manipulator 114 near the proximal end of the catheter 110 and/or deflection from the sheath 113. As shown in an inset 140, the catheter 110 can be fitted at the distal end of the shaft 112. The catheter 110 can be inserted through the sheath 113 in a collapsed state and can be then expanded within the heart 120.

Generally, electrical activity at a point in the heart 120 may be typically measured by advancing the catheter 110 containing an electrical sensor at or near its distal tip (e.g., the at least one electrode 111) to that point in the heart 120, contacting the tissue with the sensor and acquiring data at that point. One drawback with mapping a cardiac chamber using a catheter type containing only a single, distal tip electrode is the long period of time required to accumulate data on a point-by-point basis over the requisite number of points required for a detailed map of the chamber as a whole. Accordingly, multiple-electrode catheters (e.g., the catheter 110) have been developed to simultaneously measure electrical activity at multiple points in the heart chamber.

The catheter 110, which can include the at least one electrode 111 and a catheter needle coupled onto a body thereof, can be configured to obtain biometric data, such as electrical signals of an intra-body organ (e.g., the heart 120), and/or to ablate tissue areas of thereof (e.g., a cardiac chamber of the heart 120). Note that the electrodes 111 are representative of any like elements, such as tracking coils, piezoelectric transducer, electrodes, or combination of elements configured to ablate the tissue areas or to obtain the biometric data. According to one or more embodiments, the catheter 110 can include one or more position sensors that used are to determine trajectory information. The trajectory information can be used to infer motion characteristics, such as the contractility of the tissue.

Biometric data (e.g., patient biometrics, patient data, or patient biometric data) can include one or more of patient arrhythmia types, local activation times (LATs), electrical activity, topology, bipolar mapping, reference activity, ventricle activity, dominant frequency, impedance, ECG information, data at a multiplicity of points, and/or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part. ECG information can be obtained during arrhythmia treatment procedures and typically requires a significant amount of storage space and other resources to store and share this data for research. It would be beneficial to significantly reduce the need for such resources for storage and sharing of the biometric data. Also, saving biometric data obtained during cardiac electrophysiology procedures can take considerable time, e.g., hours, which can be a strain on available resources.

Examples of biometric data include, but are not limited to, patient identification data, IC ECG data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treatment any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

For example, the catheter 110 can use the electrodes 111 to implement intravascular ultrasound and/or MRI catheterization to image the heart 120 (e.g., obtain and process the biometric data). Inset 150 shows the catheter 110 in an enlarged view, inside a cardiac chamber of the heart 120. Although the catheter 110 is shown to be a point catheter, it will be understood that any shape that includes one or more elements (e.g., electrodes 111, tracking coils, piezoelectric transducer, etc.) can be used to implement the exemplary embodiments disclosed herein.

Examples of the catheter 110 include, but are not limited to, a linear catheter with multiple electrodes, a balloon catheter including electrodes dispersed on multiple spines that shape the balloon, a lasso or loop catheter with multiple electrodes, a contact-force sensing catheter, or any other applicable shape or type. Linear catheters can be fully or partially elastic such that it can twist, bend, and or otherwise change its shape based on received signal and/or based on application of an external force (e.g., cardiac tissue) on the linear catheter. The balloon catheter can be designed such that when deployed into a patient's body, its electrodes can be held in intimate contact against an endocardial surface. As an example, a balloon catheter can be inserted into a lumen, such as the PV. The balloon catheter can be inserted into the PV in a deflated state, such that the balloon catheter does not occupy its maximum volume while being inserted into the PV. The balloon catheter can expand while inside the PV, such that those electrodes on the balloon catheter are in contact with an entire circular section of the PV. Such contact with an entire circular section of the PV, or any other lumen, can enable efficient imaging and/or ablation.

According to other examples, body patches and/or body surface electrodes may also be positioned on or proximate to a body of the patient 125. The catheter 110 with the one or more electrodes 111 can be positioned within the body (e.g., within the heart 120) and a position of the catheter 110 can be determined by the 100 system based on signals transmitted and received between the one or more electrodes 111 of the catheter 110 and the body patches and/or body surface electrodes. Additionally, the electrodes 111 can sense the biometric data from within the body of the patient 125, such as within the heart 120 (e.g., the electrodes 111 sense the electrical potential of the tissue in real time). The biometric data can be associated with the determined position of the catheter 110 such that a rendering of the patient's body part (e.g., the heart 120) can be displayed and show the biometric data overlaid on a shape of the body part (i.e., mapping).

The probe 105 and other items of the system 100 can be connected to the console 160. The console 160 can include any computing device, which employs the ML/AI algorithm (represented as the mapping engine 101). According to an exemplary embodiment, the console 160 includes the one or more processors 161 (any computing hardware) and the memory 162 (any suitable non-transitory tangible, volatile, and/or non-volatile media, such as random-access memory or a hard disk drive), where the one or more processors 161 execute computer instructions with respect the mapping engine 101 and the memory 162 stores these instructions for execution by the one or more processors 161. For instance, the console 160 can be configured to receive and process the biometric data and determine if a given tissue area conducts electricity. According to one or more embodiments, the memory 162 in cooperation with the mapping engine initiate storage of details/data/information relating to an initiated medical procedure in advance of a predicted time for completion, such that storage of the details/data/information is completed following the predicted time.

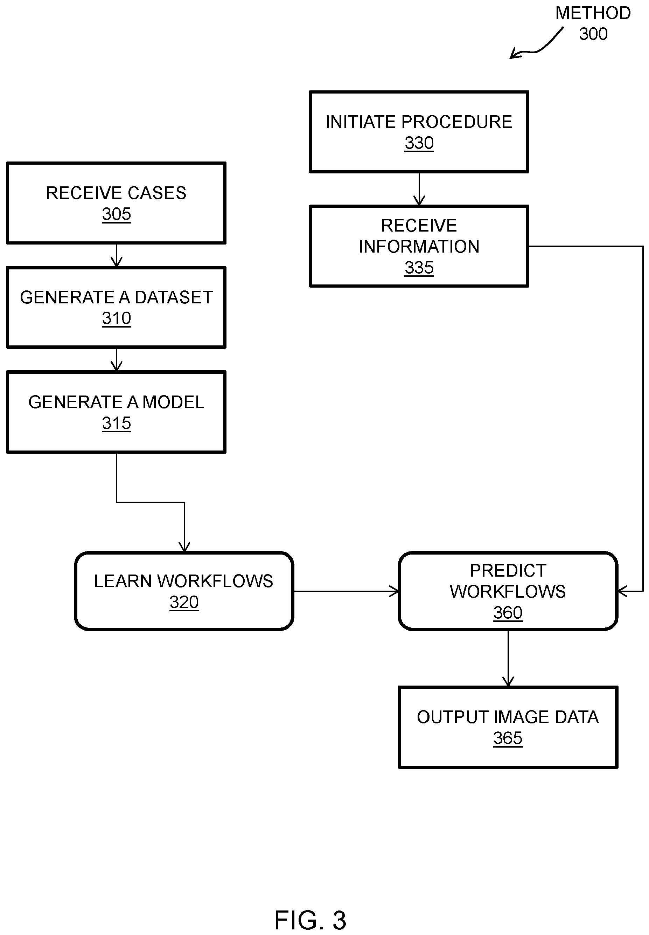

In some embodiments, the console 160 can be further programmed by the mapping engine 101 (in software) to carry out the functions described. For instance, the mapping engine 101 can include an algorithm (described herein with respect to FIG. 3), that receives biometric data acquired by the catheter 110 as it is maneuvered within the anatomical structure and provides a map. Once the map is generated, the mapping engine 101 can receive inputs representing user modifications of the map, such as through existing user interface and/or a specialized user interface of the mapping engine 101, as well as user preferences and health demographics of the patients. Generally, the mapping engine 101 can provide one or more user interfaces and intelligent toolbars, such as on behalf of the operating system or other application and/or directly, that are custom tailored based upon the biometric data, the user preferences, and the health demographics. The user interfaces and intelligent toolbars include, but are not limited to, internet browsers, graphic user interfaces (GUIs), window interfaces, toolbars, ribbons, buttons, icons, image displays and screens, pop-up windows, visualizations, templates, presentations, maps, tools, menus, and/or other visual interfaces or graphical control element inputs for applications, operating systems, file folders, and the like. As described herein, the intelligent toolbar provides buttons and options based upon physician's personal preferences obtained by ML during prior cardiac ablation treatments.

According to one or more embodiments, the mapping engine 101 can be external to the console 160 and can be located, for example, in the catheter 110, in an external device, in a mobile device, in a cloud-based device, or can be a standalone processor. In this regard, the mapping engine 101 can be transferable/downloaded in electronic form, over a network. It should be understood that mapping engine 101 can be implemented as a general purpose computer, a processor, a processor core, or fixed function circuitry, as a program, software, or firmware, stored in a non-transitory computer readable medium or in another medium, executable by a general purpose computer, a processor, or as a combination of software executing on a processor or fixed function circuitry.

In an example, the console 160 can be any computing device, as noted herein, including software (e.g., the mapping engine 101) and/or hardware (e.g., the processor 161 and the memory 162), such as a general-purpose computer, with suitable front end and interface circuits for transmitting and receiving signals to and from the probe 105, as well as for controlling the other components of the system 100. For example, the front end and interface circuits include input/output (I/O) communication interfaces that enables the console 160 to receive signals from and/or transfer signals to the at least one electrode 111. The console 160 can include real-time noise reduction circuitry typically configured as a field programmable gate array (FPGA), followed by an analog-to-digital (A/D) ECG or electrocardiograph/electromyogram (EMG) signal conversion integrated circuit. The console 160 can pass the signal from an A/D ECG or EMG circuit to another processor and/or can be programmed to perform one or more functions disclosed herein.

The display 165, which can be any electronic device for the visual presentation (e.g., via the user interfaces and the intelligent toolbars) of the biometric data, the user preferences, and the health demographics, is connected to the console 160. According to an exemplary embodiment, during a procedure, the console 160 can facilitate on the display 165 a presentation of a body part rendering to the physician 115 and store data representing the body part rendering in the memory 162. For instance, maps depicting motion characteristics can be rendered/constructed based on the trajectory information sampled at a sufficient number of points in the heart 120. As an example, the display 165 can include a touchscreen that can be configured to accept inputs from the medical professional 115, in addition to presenting the body part rendering.

In some embodiments, the physician 115 can manipulate the user interfaces, the intelligent toolbars, and/or other elements of the system 100 using one or more input devices, such as a touch pad, a mouse, a keyboard, a gesture recognition apparatus, or the like. For example, an input device can be used to change a position of the catheter 110, such that rendering is updated. Note that the display 165 can be located at a same location or a remote location, such as a separate hospital or in separate healthcare provider networks.

According to one or more embodiments, the system 100 can also obtain the biometric data using ultrasound, computed tomography (CT), MRI, or other medical imaging techniques utilizing the catheter 110 or other medical equipment. For instance, the system 100 can obtain ECG data and/or anatomical and electrical measurements of the heart 120 (e.g., the biometric data) using one or more catheters 110 or other sensors. More particularly, the console 160 can be connected, by a cable, to BS electrodes, which include adhesive skin patches affixed to the patient 125. The BS electrodes can procure/generate the biometric data in the form of the BS ECG data. For instance, the processor 161 can determine position coordinates of the catheter 110 inside the body part (e.g., the heart 120) of the patient 125. The position coordinates may be based on impedances or electromagnetic fields measured between the body surface electrodes and the electrode 111 of the catheter 110 or other electromagnetic components. Additionally, or alternatively, location pads, which generate magnetic fields used for navigation, may be located on a surface of the bed 130 and may be separate from the bed 130. The biometric data can be transmitted to the console 160 and stored in the memory 162. Alternatively, or in addition, the biometric data (as well as the user preferences and the health demographics) may be transmitted to a server, which may be local or remote, using a network as further described herein.

According to one or more exemplary embodiments, the catheter 110 may be configured to ablate tissue areas of a cardiac chamber of the heart 120. Inset 150 shows the catheter 110 in an enlarged view, inside a cardiac chamber of the heart 120. For instance, ablation electrodes, such as the at least one electrode 111, may be configured to provide energy to tissue areas of an intra-body organ (e.g., the heart 120). The energy may be thermal energy and may cause damage to the tissue area starting from the surface of the tissue area and extending into the thickness of the tissue area. The biometric data with respect to ablation procedures (e.g., ablation tissues, ablation locations, etc.) can be considered ablation data.

According to an example, with respect to obtaining the biometric data, a multi-electrode catheter (e.g., the catheter 110) can be advanced into a chamber of the heart 120. Anteroposterior (AP) and lateral fluorograms can be obtained to establish the position and orientation of each of the electrodes. ECGs can be recorded from each of the electrodes 111 in contact with a cardiac surface relative to a temporal reference, such as the onset of the P-wave in sinus rhythm from a BS ECG and/or signals from electrodes 111 of the catheter 110 placed in the coronary sinus. The system, as further disclosed herein, may differentiate between those electrodes that register electrical activity and those that do not due to absence of close proximity to the endocardial wall. After initial ECGs are recorded, the catheter may be repositioned, and fluorograms and ECGs may be recorded again. An electrical map (e.g., via cardiac mapping) can then be constructed from iterations of the process above.

Cardiac mapping can be implemented using one or more techniques. Generally, mapping of cardiac areas such as cardiac regions, tissue, veins, arteries and/or electrical pathways of the heart 120 may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to LAT, local activation velocity, an electrical activity, a topology, a bipolar mapping, a dominant frequency, or an impedance. Data (e.g., biometric data) corresponding to multiple modalities may be captured using a catheter (e.g., the catheter 110) inserted into a patient's body and may be provided for rendering at the same time or at different times based on corresponding settings and/or preferences of the physician 115.

As an example of a first technique, cardiac mapping may be implemented by sensing an electrical property of heart tissue, for example, LAT, as a function of the precise location within the heart 120. The corresponding data (e.g., biometric data) may be acquired with one or more catheters (e.g., the catheter 110) that are advanced into the heart 1120 and that have electrical and location sensors (e.g., the electrodes 111) in their distal tips. As specific examples, location and electrical activity may be initially measured on about 10 to about 20 points on the interior surface of the heart 120. These data points may be generally sufficient to generate a preliminary reconstruction or map of the cardiac surface to a satisfactory quality. The preliminary map may be combined with data taken at additional points to generate a more comprehensive map of the heart's electrical activity. In clinical settings, it is not uncommon to accumulate data at 100 or more sites (e.g., several thousand) to generate a detailed, comprehensive map of heart chamber electrical activity. The generated detailed map may then serve as the basis for deciding on a therapeutic course of action, for example, tissue ablation as described herein, to alter the propagation of the heart's electrical activity and to restore normal heart rhythm.

Further, cardiac mapping can be generated based on detection of intracardiac electrical potential fields (e.g., which is an example of IC ECG data and/or bipolar intracardiac reference signals). A non-contact technique to simultaneously acquire a large amount of cardiac electrical information may be implemented. For example, a catheter type having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes). As another more specific example, the catheter may include other multi-spline catheters, such as five soft flexible branches, eight radial splines, or a parallel splined pancake turner type (e.g., any of which may have a total of 42 electrodes).

As example of electrical or cardiac mapping, an electrophysiological cardiac mapping system and technique based on a non-contact and non-expanded multi-electrode catheter (e.g., the catheter 110) can be implemented. ECGs may be obtained with one or more catheters 110 having multiple electrodes (e.g., such as between 42 to 122 electrodes). According to this implementation, knowledge of the relative geometry of the probe and the endocardium can be obtained by an independent imaging modality, such as transesophageal echocardiography. After the independent imaging, non-contact electrodes may be used to measure cardiac surface potentials and construct maps therefrom (e.g., in some cases using bipolar intracardiac reference signals). This technique can include the following steps (after the independent imaging step): (a) measuring electrical potentials with a plurality of electrodes disposed on a probe positioned in the heart 120; (b) determining the geometric relationship of the probe surface and the endocardial surface and/or other reference; (c) generating a matrix of coefficients representing the geometric relationship of the probe surface and the endocardial surface; and (d) determining endocardial potentials based on the electrode potentials and the matrix of coefficients.

As another example of electrical or cardiac mapping, a technique and apparatus for mapping the electrical potential distribution of a heart chamber can be implemented. An intra-cardiac multi-electrode mapping catheter assembly can be inserted into the heart 120. The mapping catheter (e.g., the catheter 110) assembly can include a multi-electrode array with one or more integral reference electrodes (e.g., one or the electrodes 111) or a companion reference catheter.

According to one or more exemplary embodiments, the electrodes may be deployed in the form of a substantially spherical array, which may be spatially referenced to a point on the endocardial surface by the reference electrode or by the reference catheter this is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

In view of electrical or cardiac mapping and according to another example, the catheter 110 can be a heart mapping catheter assembly that may include an electrode array defining a number of electrode sites. The heart mapping catheter assembly can also include a lumen to accept a reference catheter having a distal tip electrode assembly that may be used to probe the heart wall. The map heart mapping catheter assembly can include a braid of insulated wires (e.g., having 24 to 64 wires in the braid), and each of the wires may be used to form electrode sites. The heart mapping catheter assembly may be readily positionable in the heart 120 to be used to acquire electrical activity information from a first set of non-contact electrode sites and/or a second set of in-contact electrode sites.

Further, according to another example, the catheter 110 that can implement mapping electrophysiological activity within the heart can include a distal tip that is adapted for delivery of a stimulating pulse for pacing the heart or an ablative electrode for ablating tissue in contact with the tip. This catheter 110 can further include at least one pair of orthogonal electrodes to generate a difference signal indicative of the local cardiac electrical activity adjacent the orthogonal electrodes.

As noted herein, the system 100 can be utilized to detect, diagnose, and/or treat cardiac conditions. In example operation, a process for measuring electrophysiologic data in a heart chamber may be implemented by the system 100. The process may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred embodiments, the array is said to have from 60 to 64 electrodes.

As another example operation, cardiac mapping may be implemented by the system 100 using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of the probe 105 (e.g., a treatment catheter shown as catheter 110) at the later time may be displayed and the probe 105 may be overlaid onto the one or more ultrasound slices.

In view of the system 100, it is noted that cardiac arrhythmias, including atrial arrhythmias, may be of a multiwavelet reentrant type, characterized by multiple asynchronous loops of electrical impulses that are scattered about the atrial chamber and are often self-propagating (e.g., another example of the IC ECG data). Alternatively, or in addition to the multiwavelet reentrant type, cardiac arrhythmias may also have a focal origin, such as when an isolated region of tissue in an atrium fires autonomously in a rapid, repetitive fashion (e.g., another example of the IC ECG data). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

For example, aFib occurs when the normal electrical impulses (e.g., another example of the IC ECG data) generated by the sinoatrial node are overwhelmed by disorganized electrical impulses (e.g., signal interference) that originate in the atria veins and PVs causing irregular impulses to be conducted to the ventricles. An irregular heartbeat results and may last from minutes to weeks, or even years. aFib is often a chronic condition that leads to a small increase in the risk of death often due to strokes. A line of treatment for aFib is medication that either slows the heart rate or revert the heart rhythm back to normal. Additionally, persons with aFib are often given anticoagulants to protect them from the risk of stroke. The use of such anticoagulants comes with its own risk of internal bleeding. In some patients, medication is not sufficient and their aFib is deemed to be drug-refractory, i.e., untreatable with standard pharmacological interventions. Synchronized electrical cardioversion may also be used to convert aFib to a normal heart rhythm. Alternatively, aFib patients are treated by catheter ablation.

A catheter ablation-based treatment may include mapping the electrical properties of heart tissue, especially the endocardium and the heart volume, and selectively ablating cardiac tissue by application of energy. Electrical or cardiac mapping (e.g., implemented by any electrophysiological cardiac mapping system and technique described herein) includes creating a map of electrical potentials (e.g., a voltage map) of the wave propagation along the heart tissue or a map of arrival times (e.g., a LAT map) to various tissue located points. Electrical or cardiac mapping (e.g., a cardiac map) may be used for detecting local heart tissue dysfunction. Ablations, such as those based on cardiac mapping, can cease or modify the propagation of unwanted electrical signals from one portion of the heart 120 to another.

The ablation process damages the unwanted electrical pathways by formation of non-conducting lesions. Various energy delivery modalities have been disclosed for forming lesions, and include use of microwave, laser and more commonly, RF energies to create conduction blocks along the cardiac tissue wall. Another example of an energy delivery technique includes irreversible electroporation (IRE), which provides high electric fields that damage cell membranes. In a two-step procedure (e.g., mapping followed by ablation) electrical activity at points within the heart 120 is typically sensed and measured by advancing the catheter 110 containing one or more electrical sensors (e.g., electrodes 111) into the heart 120 and obtaining/acquiring data at a multiplicity of points (e.g., as biometric data generally). The biometric data is then utilized to select the endocardial target areas, at which ablation is to be performed. Note that, due to the use of the mapping engine 101 employed by the exemplary system 100 (e.g., medical device equipment), the data at the multiplicity of points is manipulated into improved image data of cardiac tissue.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems to reconstruct the anatomy of the heart chamber of interest. In this regard, the mapping engine 101 employed by the system 100 herein manipulates and evaluates the biometric data generally to produce improved tissue data that enables more accurate diagnosis, images, scans, and/or maps for treating an abnormal heartbeat or arrhythmia. For example, cardiologists rely upon software, such as the Complex Fractionated Atrial Electrograms (CFAE) module of the CARTO® 3 3D mapping system, produced by Biosense Webster, Inc. (Diamond Bar, Calif.), to generate and analyze ECG data. The mapping engine 101 of the system 100 enhances this software to generate and analyze the improved biometric data, which further provide multiple pieces of information regarding electrophysiological properties of the heart 120 (including the scar tissue) that represent cardiac substrates (anatomical and functional) of aFib.

Accordingly, the system 100 can implement a 3D mapping system, such as CARTO® 3 3D mapping system, to localize the potential arrhythmogenic substrate of the cardiomyopathy in terms of abnormal ECG detection. The substrate linked to these cardiac conditions is related to the presence of fragmented and prolonged ECGs in the endocardial and/or epicardial layers of the ventricular chambers (right and left). For instance, areas of low or medium voltage may exhibit ECG fragmentation and prolonged activities. Further, during sinus rhythm, areas of low or medium voltage may corresponds to a critical isthmus identified during sustained and organized ventricular arrhythmias (e.g., applies to non-tolerated ventricular tachycardias, as well as in the atria). In general, abnormal tissue is characterized by low-voltage ECGs. However, initial clinical experience in endo-epicardial mapping indicates that areas of low-voltage are not always present as the sole arrhythmogenic mechanism in such patients. In fact, areas of low or medium voltage may exhibit ECG fragmentation and prolonged activities during sinus rhythm, which corresponds to the critical isthmus identified during sustained and organized ventricular arrhythmias, e.g., applies only to non-tolerated ventricular tachycardias. Moreover, in many cases, ECG fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

As another example operation, cardiac mapping may be implemented by the system 100 using one or more multiple-electrode catheters (e.g., the catheter 110). Multiple-electrode catheters are used to stimulate and map electrical activity in the heart 120 and to ablate sites of aberrant electrical activity. In use, the multiple-electrode catheter is inserted into a major vein or artery, e.g., femoral vein, and then guided into the chamber of the heart 120 of concern. A typical ablation procedure involves the insertion of the catheter 110 having at least one electrode 111 at its distal end, into a heart chamber. A reference electrode is provided, taped to the skin of the patient or by means of a second catheter that is positioned in or near the heart or selected from one or the other electrodes 111 of the catheter 110. Radio frequency (RF) current is applied to a tip electrode 111 of the ablating catheter 110, and current flows through the media that surrounds it (e.g., blood and tissue) toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the tip electrode 111 also occurs as a result of conduction from the heated tissue to the electrode itself. If the electrode temperature becomes sufficiently high, possibly above 60 degrees Celsius, a thin transparent coating of dehydrated blood protein can form on the surface of the electrode 111. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter 110 must be removed from the body and the tip electrode 111 cleaned.

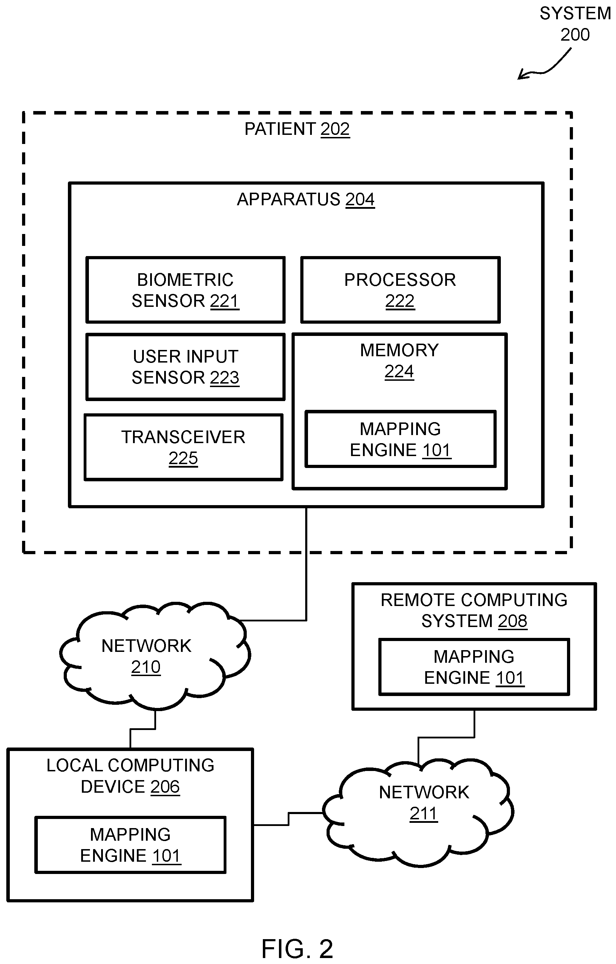

Turning now to FIG. 2, a diagram of a system 200 in which one or more features of the disclosure subject matter can be implemented is illustrated according to one or more exemplary embodiments. The system 200 includes, in relation to a patient 202 (e.g., an example of the patient 125 of FIG. 1), an apparatus 204, a local computing device 206, a remote computing system 208, a first network 210, and a second network 211. Further, the apparatus 204 can include a biometric sensor 221 (e.g., an example of the catheter 110 of FIG. 1), a processor 222, a user input (UI) sensor 223, a memory 224, and a transceiver 225. Note that the mapping engine 101 of FIG. 1 is reused in FIG. 2 for ease of explanation and brevity.

According to an embodiment, the apparatus 204 can be an example of the system 100 of FIG. 1, where the apparatus 204 can include both components that are internal to the patient and components that are external to the patient. The apparatus 204 may also include a catheter with one or more electrodes (e.g., the catheter 110 and the electrodes 111 of FIG. 1), a probe (e.g., the probe 105 of FIG. 1), a blood pressure cuff, a weight scale, a bracelet or smart watch biometric tracker, a glucose monitor, a continuous positive airway pressure (CPAP) machine or virtually any device which may provide an input concerning the health or biometrics of the patient 202 (e.g., the patient 125 of FIG. 1). According to another embodiment, the apparatus 204 can be an apparatus that is external to the patient 202 that includes an attachable patch (e.g., that attaches to a patient's skin). According to another embodiment, the apparatus 204 can be internal to a body of the patient 202 (e.g., subcutaneously implantable), where the apparatus 204 can be inserted into the patient 202 via any applicable manner including orally injecting, surgical insertion via a vein or artery, an endoscopic procedure, or a laparoscopic procedure. According to an embodiment, while a single apparatus 204 is shown in FIG. 2, example systems may include a plurality of apparatuses.

Accordingly, the apparatus 204, the local computing device 206, and/or the remote computing system 208 can be programed to execute computer instructions with respect the mapping engine 101. As an example, the memory 223 stores these instructions for execution by the processor 222 so that the apparatus 204 can receive and process the biometric data via the biometric sensor 201. In this way, the processor 222 and the memory 223 are representative of processors and memories of the local computing device 206 and/or the remote computing system 208.

The apparatus 204, the local computing device 206, and/or the remote computing system 208 can be any combination of software and/or hardware that individually or collectively store, execute, and implement the mapping engine 101 and functions thereof. Further, the apparatus 204, local computing device 206, and/or the remote computing system 208 can be an electronic, computer framework comprising and/or employing any number and combination of computing device and networks utilizing various communication technologies, as described herein. The apparatus 204, local computing device 206, and/or the remote computing system 208 can be easily scalable, extensible, and modular, with the ability to change to different services or reconfigure some features independently of others.

The networks 210 and 211 can be a wired network, a wireless network, or include one or more wired and wireless networks. According to an embodiment, the network 210 is an example of a short-range network (e.g., local area network (LAN), or personal area network (PAN)). Information can be sent, via the network 210, between the apparatus 204 and the local computing device 206 using any one of various short-range wireless communication protocols, such as Bluetooth, Wi-Fi, Zigbee, Z-Wave, near field communications (NFC), ultra-band, Zigbee, or infrared (IR). Further, the network 211 is an example of one or more of an Intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information can be sent, via the network 211, using any one of various long-range wireless communication protocols (e.g., TCP/IP, HTTP, 3G, 4G/LTE, or 5G/New Radio). Note that for either network 210 and 211 wired connections can be implemented using Ethernet, Universal Serial Bus (USB), RJ-11 or any other wired connection and wireless connections can be implemented using Wi-Fi, WiMAX, and Bluetooth, infrared, cellular networks, satellite or any other wireless connection methodology.

In operation, the apparatus 204 can continually or periodically obtain, monitor, store, process, and communicate via network 210 the biometric data associated with the patient 202. Further, the apparatus 204, local computing device 206, and/ the remote computing system 208 are in communication through the networks 210 and 211 (e.g., the local computing device 206 can be configured as a gateway between the apparatus 204 and the remote computing system 208). For instance, the apparatus 204 can be an example of the system 100 of FIG. 1 configured to communicate with the local computing device 206 via the network 210. The local computing device 206 can be, for example, a stationary/standalone device, a base station, a desktop/laptop computer, a smart phone, a smartwatch, a tablet, or other device configured to communicate with other devices via networks 211 and 210. The remote computing system 208, implemented as a physical server on or connected to the network 211 or as a virtual server in a public cloud computing provider (e.g., Amazon Web Services (AWS) ®) of the network 211, can be configured to communicate with the local computing device 206 via the network 211. Thus, the biometric data associated with the patient 202 can be communicated throughout the system 200.

Elements of the apparatus 204 are now described. The biometric sensor 221 may include, for example, one or more transducers configured to convert one or more environmental conditions into an electrical signal, such that different types of biometric data are observed/obtained/acquired. For example, the biometric sensor 221 can include one or more of an electrode (e.g., the electrode 111 of FIG. 1), a temperature sensor (e.g., thermocouple), a blood pressure sensor, a blood glucose sensor, a blood oxygen sensor, a pH sensor, an accelerometer, and a microphone.

The processor 222, in executing the mapping engine 101, can be configured to receive, process, and manage the biometric data acquired by the biometric sensor 221, and communicate the biometric data, the user preferences, and the health demographics to the memory 224 for storage and/or across the network 210 via the transceiver 225. As described in more detail herein, the mapping engine 101 produces improved image data of cardiac tissue so that improved images, scans, and/or maps, along with predictions, for treating cardiac conditions can be generated.

The biometric data, the user preferences, and the health demographics from one or more other apparatuses 204 can also be received by the processor 222 through the transceiver 225. Also, as described in more detail herein, the processor 222 may be configured to respond selectively to different tapping patterns (e.g., a single tap or a double tap) received from the UI sensor 223, such that different tasks of a patch (e.g., acquisition, storing, or transmission of data) can be activated based on the detected pattern. In some embodiments, the processor 222 can generate audible feedback with respect to detecting a gesture.

The UI sensor 223 includes, for example, a piezoelectric sensor or a capacitive sensor configured to receive a user input, such as a tapping or touching. For example, the UI sensor 223 can be controlled to implement a capacitive coupling, in response to tapping or touching a surface of the apparatus 204 by the patient 202. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface, such that the tapping or touching of the surface activates the monitoring device.

The memory 224 is any non-transitory tangible media, such as magnetic, optical, or electronic memory (e.g., any suitable volatile and/or non-volatile memory, such as random-access memory or a hard disk drive). The memory 224 stores the computer instructions for execution by the processor 222 and/or the biometric data, the user preferences, and the health demographics. Examples of user preferences include, but at not limited to, voltage selections, threshold selection, font size, interface locations, button configurations, and color scheme. Examples of health demographics include, but at not limited to, age, gender, race, weight, ethnicity, and height.

The transceiver 225 may include a separate transmitter and a separate receiver. Alternatively, the transceiver 225 may include a transmitter and receiver integrated into a single device.

In operation, the apparatus 204, utilizing the mapping engine 101, observes/obtains the biometric data of the patient 202 via the biometric sensor 221; observes/obtains the user preferences and the health demographics; stores the biometric data, the user preferences, and the health demographics in the memory 224, and shares this biometric data across the system 200 via the transceiver 225. The mapping engine 101 can then utilize models, algorithms, neural networks, ML, and/or artificial intelligence (AI) to generate and provide mappings to physician, to reduce processing loads for the system 100, and to transform operations of the system 100 to provide more accurate mappings.

Turning now to FIG. 3, a method 300 (e.g., performed by the mapping engine 101 of FIG. 1 and/or of FIG. 2) is illustrated according to one or more exemplary embodiments. Any combination of software and/or hardware (e.g., the local computing device 206 and the remote computing system 208, along with the apparatus 204) can individually or collectively store, execute, and implement the mapping engine 101 and functions thereof. The method 300 addresses the shortcomings of conventional cardiac mapping and ablation systems.

In general, the physician 115 conducts a present cardiac electrophysiology procedure using the system 100 on the patient 125. The cardiac electrophysiology procedure, such as an arrhythmia therapeutic procedure, can be any medical and/or surgical procedure or treatment on the patient. The mapping engine 101 receives past workflow preferences exhibited by the physician 115, along with the unique physiological arrhythmia characteristics (e.g., the biometric data) and the health demographics. Workflow preferences include choices in steps (e.g., step A instead of B), order of steps (e.g., step B instead A), choice in equipment, choice in user interface, setting of a user interface, etc. The mapping engine 101 then learns these past workflow preferences, with the unique physiological arrhythmia characteristics and the health demographics, to predict image displays to present to the physician 115 during the present cardiac electrophysiology procedure. One or more technical effects and benefits of the predicted image displays (e.g., one or more user interfaces and intelligent toolbars) include reducing procure time and streamlining workflow.

The process flow 300 begins at block 305, where the mapping engine 101 receives cases. The cases can include past medical procedures and/or treatments (e.g., past cardiac electrophysiology procedure). In this regard, the mapping engine 101 can receive source data, atomic data, or primary data of the cases. In one or more embodiments, the source data, atomic data, or primary data can be qualified and quantified as historical data for the past medical procedures and/or treatments on a plurality of patient.

The historical data can include workflow preferences of one or more users (e.g., the physician 115 and/or technician) performing these past medical procedures and/or treatments, as well as the biometric data and/or health demographics of the plurality of patients of the plurality of patients. The biometric data can include unique physiological characteristics of one or more arrhythmias of the patient.

According to one or more embodiments, the historical data can included a first set of information regarding user events obtained from prior cardiac electrophysiology procedures, a second set of information regarding sensor events obtained from prior cardiac electrophysiology procedures, a third set of information including hybrid events obtained from prior cardiac electrophysiology procedures, details relating to patient setup, details relating to removal of catheters from the patient at the conclusion of the procedure, details of interest to the physician, and other details relating to the cardiac electrophysiology procedure itself.

At block 310, the mapping engine 101 generates a dataset. The dataset can be generated and/or built by the mapping engine 101 by collecting and manipulating raw data (e.g., source data, atomic data or primary data from block 305), whether organized and/or disorganized, in one or more data structures (e.g., data organization, management, and storage format that enables efficient access and modification). The dataset can be created, used, and maintained as a training data for training and ML/AI.

According to one or more embodiments, the mapping engine 101 builds the training data to easily organize and analyze the historical data, as well as other data associated therewith (e.g., types of past medical procedures and/or treatments, injury and/or disease diagnosis, health demographic information for the plurality of patients, physician information, technician information, procedure and/or treatment location information, annotated anatomical structures of a cardiac tissue information, etc.).

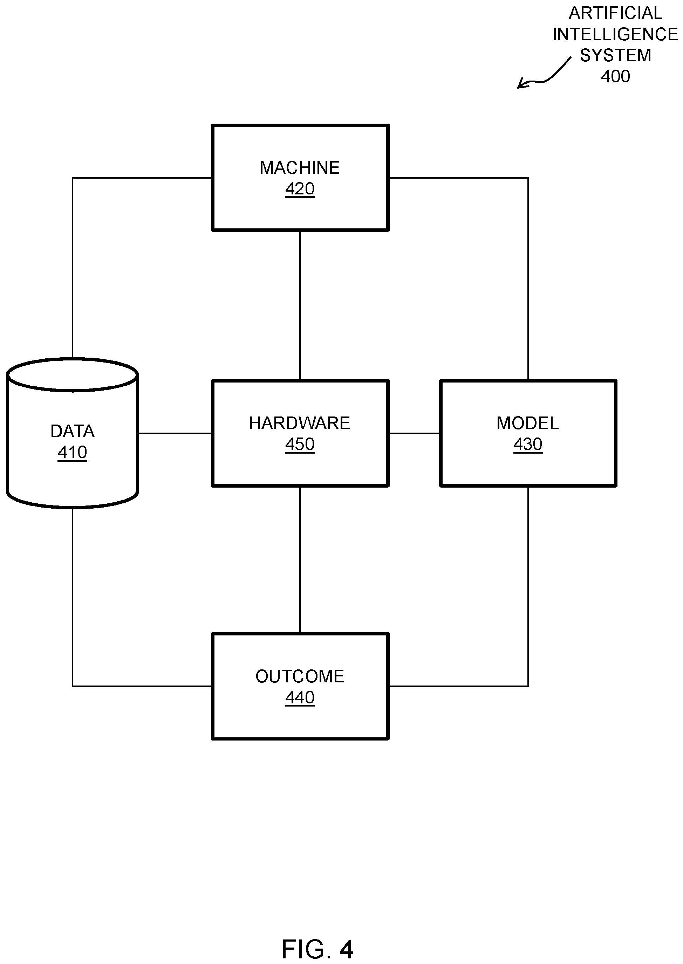

With respect to ML/AI by the system 100 and the mapping engine 101 at various blocks of FIG. 3, FIGS. 4 and 5 are provided to define examples thereof. The description of FIGS. 4-5 is made with reference to FIGS. 1-3 for ease of understanding where appropriate. FIG. 4 illustrates a graphical depiction of an artificial intelligence system 400 according to one or more embodiments. The artificial intelligence system 400 includes data 410 (e.g., the biometric data, the historical data, etc. of block 310), a machine 420, a model 430, an outcome 440, and (underlying) hardware 450. For example, the machine 410, the model 430, and the hardware 450 can represent aspects of the mapping engine 101 of FIGS. 1-2 (e.g., ML/AI algorithm therein), while the hardware 450 can also represent the catheter 110 of FIG. 1, the console 160 of FIG. 1, and/or the apparatus 204 of FIG. 2. In general, the ML/AI algorithms of the artificial intelligence system 400 (e.g., as implemented by the mapping engine 101 of FIGS. 1-2) operate with respect to the hardware 450, using the data 410 (e.g., of block 310), to train the machine 420, build the model 430, and predict the outcomes 440. Further, it should be understood that mapping engine 101 can be implemented as or with respect to the data 410, the machine 420, the model 430, the outcome 440, and any (underlying) hardware 450.

For instance, with respect to block 310, the machine 420 operates as the controller or data collection associated with the hardware 450 and/or is associated therewith. The data 410 (e.g., the biometric data, the historical data, etc. of block 310) can be on-going data or output data associated with the hardware 450. The data 410 can also include currently collected data, training data, and/or other information of a received case (e.g., block 305) from the hardware 450; can include measurements during a surgical procedure and may be associated with an outcome of the surgical procedure; can include a temperature of the heart 140 of FIG. 1 collected and correlated with an outcome of a heart procedure; can include past workflow preferences; can include ablation information and determinations as to whether redo procedures are required; and can be related to the hardware 450. The data 410 can be divided by the machine 420 into one or more subsets.

More particularly with respect to FIG. 3, in accordance with one or more embodiments, training data may include user specific preferences, workflows, annotations, aspects of the historical data, etc. In this regard, the mapping engine 101 collects and organizes (across blocks 305 and 310) information representing user decisions and modifications as well as automatic decisions and suggestions made by the mapping engine 101 during the past medical procedures and/or treatments and user inputs responsive to these automatic decisions (e.g., a physician 115 manually changes a default preference setting) and suggestions (e.g., the physician 115 manually changes a map suggestion) to provide a baseline. In turn, if/when the method 300 repeats or loops, the training dataset can increase and/or improve over time (e.g., on average every 3 - 6 months) and can be used to train/retrain the mapping engine 101 (e.g., to improve a performance thereof).

At block 315, the mapping engine 101 generates a model (e.g., the 430 of FIG. 4) of the mapping engine 101. The model 430 can be a ML/AI algorithm. The model 430 can include a trained deep learning architecture using a recurrent neural network (RNN). The model 340 is built and trained based upon analysis of a plurality of prior cardiac electrophysiology cases, such as described by the dataset (or more specifically, the training data) of block 310.

In the FIG. 4 example, the model 430 is built on the data 410 associated with the hardware 450. Then, the model 430 and the machine 420 can train with respect to the hardware 450. Building the model 430 can include physical hardware or software modeling, algorithmic modeling (block 315), and/or the like that seeks to represent the data 410 (or subsets thereof) that has been collected and trained. In some aspects, building of the model 430 (block 315) is part of self-training operations by the machine 420. This training can include an analysis and correlation of the data 410 collected. For example, the machine 420 may be trained to determine if a correlation or link exists between the biometric data, the user preferences, and/or the health demographics. In accordance with another embodiment, training the machine 420 can include self-training by the mapping engine 101 of FIG. 1 utilizing the one or more subsets. In this regard, the mapping engine 101 of FIG. 1 learns to workflows, medical procedure workflow preferences, the unique physiological characteristics, and/or the personal health demographics. Thus, the model 430 can be configured to replicate the operation of hardware 450 and replicate the data 410 collected (block 305) from the hardware 450 to predict the outcome 440 achieved by the hardware 450.

Returning to FIG. 3, at block 320, the mapping engine 101 learns one or more workflows. The mapping engine 101 can utilize the model (block 315) to learn workflows during past medical procedures and/or treatments (e.g., cardiac arrhythmia treatments) based upon particular preferences of physicians carrying out the procedure and treatments, types of arrhythmias to be treated, and patient demographics. As related to FIG. 4, the model 430, with respect to the mapping engine 101 of FIG. 1, may receive the data 410 (block 305), estimate an intermediate dataset (block 310), generate the model 430 (block 315), and provide a result (e.g., learned workflows at block 320) to support treatment of cardiac tissue, conditions, and concerns. The operation of predicting the outcomes 440 (of the model 430 associated with the hardware 450) can utilize a trained model. Thus, using the outcome 440 that is predicted, the machine 420, the model 430, and the hardware 450 can be configured accordingly.

According to one or more embodiments, when/after the mapping engine 101 generates and trains (block 315) the model using the historical data (of blocks 305 and 310), ML of the mapping engine 101 selects, identifies, and saves (block 320) only those details of interest to the physician 115 (e.g., CAS, user, or the like). In an example, the physician's personal preferences can be obtained and identified by the mapping engine 101 during prior cardiac ablation treatments. That is, during arrhythmia treatments, each time the physician 115 clicks on a button (e.g., "SWITCH TO BIPOLAR MAP", "POINT ACQUISITION", "FILTER SETUP", etc.), the button click and related information is saved as data. Also, contextual events, such as "ABLATION STARTED", "ABLATION ENDED", "SIMULATION STARTED", "STIMULATION ENDED", changes in the cardiac cycle length, changes in the arrhythmia are saved. The mapping engine 101 can utilize the model (block 315) to learn follows up clicks and adds items to the intelligent toolbar accordingly, and/or that a probability that the physician 115 presses a "NEW MAP" button is high after a change in the cycle length.

Generally, the model of the mapping engine 101 (or the model 430) can be structured as an autoencoder including an input layer, a hidden layer, and an output layer, where the input layer and the hidden layer form an encoder for encoding inputs (e.g., receives cases) and the hidden layer and the output layer form a decoder for decoding a result of the encoder. Thus, the hidden layer is disposed between the input layer and the output layer, while the inputs can include at least signal obtained from the catheter 110 and compressed by the system 100 to reduce storage resource needs. As discussed herein, the encoder can be arranged to receive the inputs at the input layer. The hidden layer can utilize a first neural network to compress the inputs to create a representation thereof at a predetermined compression ratio, can utilize a second neural network to decompress the representation of the inputs to reconstruct the inputs, and can send the reconstruction to the output layer of the decoder. The first and/or second neural networks can be convolutional and de-convolutional neural networks, or any other neural network described herein.

In general, a neural network is a network or circuit of neurons, or in a modern sense, an artificial neural network (ANN), composed of artificial neurons or nodes or cells. According to one or more embodiments, a neural network is an auto-completion ML technology, such as an RNN, with a subtype of RNNs being long short-term memory (LSTM) neural networks. As further described herein, the mapping engine 101 and/or the machine 420 can include RNN AI transformers as described herein. For example, with respect to FIG. 4, the ML/AI algorithms of the artificial intelligence system 400 can include neural networks to operate with respect to the hardware 450, to use the data 410, to train the machine 420, to build the model 430, and to predict the outcomes 440.

For example, an ANN involves a network of processing elements (artificial neurons) which can exhibit complex global behavior, determined by the connections between the processing elements and element parameters. These connections of the network or circuit of neurons are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1. In most cases, the ANN is an adaptive system that changes its structure based on external or internal information that flows through the network.

In more practical terms, neural networks are non-linear statistical data modeling or decision-making tools that can be used to model complex relationships between inputs and outputs or to find patterns in data. Thus, ANNs or RNNs may be used for predictive modeling and adaptive control applications, while being trained via a dataset. Note that self-learning resulting from experience can occur within ANNs or RNNs which can derive conclusions from a complex and seemingly unrelated set of information. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data (e.g., the biometric data) or task (e.g., monitoring, diagnosing, and treating any number of various diseases) makes the design of such functions by hand impractical.

Neural networks can be used in different fields. Thus, for the artificial intelligence system 400, the ML/AI algorithms therein can include neural networks that are divided generally according to tasks to which they are applied. These divisions tend to fall within the following categories: regression analysis (e.g., function approximation) including time series prediction and modeling; classification including pattern and sequence recognition; novelty detection and sequential decision making; data processing including filtering; clustering; blind signal separation, and compression. For example, Application areas of ANNs or RNNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis and treatment, financial applications, data mining (or knowledge discovery in databases, "KDD"), visualization and e-mail spam filtering. For example, it is possible to create a semantic profile of patient biometric data emerging from medical procedures.

According to one or more embodiments, the neural network can implement a LSTM neural network architecture, a convolutional neural network (CNN) architecture, or other the like. The neural network can be configurable with respect to a number of layers, a number of connections (e.g., encoder/decoder connections), a regularization technique (e.g., dropout); and an optimization feature.

The LSTM neural network architecture includes feedback connections and can process single data points (e.g., such as images), along with entire sequences of data (e.g., such as speech or video). A unit of the LSTM neural network architecture can be composed of a cell, an input gate, an output gate, and a forget gate, where the cell remembers values over arbitrary time intervals and the gates regulate a flow of information into and out of the cell.

The CNN architecture is a shared-weight architecture with translation invariance characteristics where each neuron in one layer is connected to all neurons in the next layer. The regularization technique of the CNN architecture can take advantage of the hierarchical pattern in data and assemble more complex patterns using smaller and simpler patterns. If the neural network implements the CNN architecture, other configurable aspects of the architecture can include a number of filters at each stage, kernel size, a number of kernels per layer.

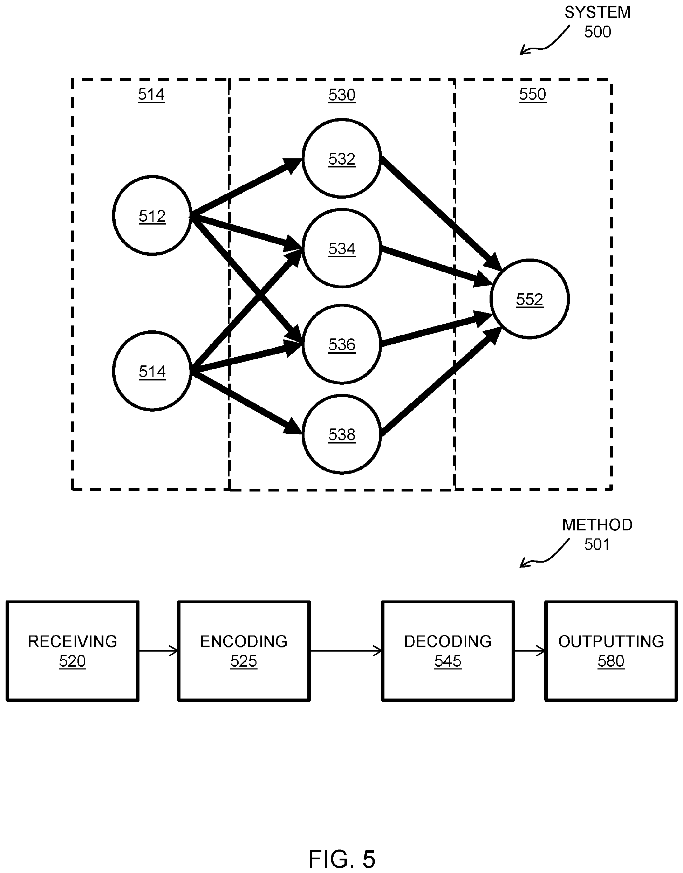

Turning now to FIG. 5, an example of a neural network 500 and a block diagram of a method 501 performed in the neural network 500 are shown according to one or more embodiments. The neural network 500 operates to support implementation of the ML/AI algorithms (e.g., as implemented by the mapping engine 101 of FIGS. 1-2) described herein.

The neural network 500 can be implemented in hardware, such as the machine 420 and/or the hardware 450 of FIG. 4. Note that, according to one or more embodiments, the neural network 500 can be implemented in hardware and/or software. According to one or more embodiments, the neural network 600 can be an autoencoder of the mapping engine 101 that extracts intracardiac based features for classification. As indicated herein, the description of FIGS. 4-5 is made with reference to FIGS. 1-3 for ease of understanding where appropriate.

In an example operation, the mapping engine 101 of FIG. 1 includes collecting the data 410 from the hardware 450. In the neural network 500, an input layer 510 is represented by a plurality of inputs (e.g., inputs 512 and 514 of FIG. 5). With respect to block 520 of the method 501, the input layer 510 receives the inputs 512 and 514. The inputs 512 and 514 can include biometric data (e.g., arrhythmia types), the user preferences, and the health demographics. For example, the collecting of the data 410 can be an aggregation of biometric data (e.g., BS ECG data, IC ECG data, and ablation data, along with catheter electrode position data), from one or more procedure recordings or cases of the hardware 450 into a dataset (as represented by the data 410). The plurality of inputs can be ultrasound signals, radio signals, audio signal, or a two- or three-dimensional image. More particularly, the plurality of inputs can be represented as input data (X), which is raw data recorded from, for example, a heart mapping phase.

At block 525 of the method 501, the neural network 500 encodes the inputs 512 and 514 utilizing any portion of the data 410 (e.g., the dataset and predictions produced by the artificial intelligence system 400) to produce a latent representation or data coding. The latent representation includes one or more intermediary data representations derived from the plurality of inputs. According to one or more embodiments, the latent representation is generated by an element-wise activation function (e.g., a sigmoid function or a rectified linear unit) of the autoencoder of the mapping engine 101 that applies a weight matrix to the input intracardiac signals and adds a bias vector to the result. Note that weights and biases of the weight matrix and the bias vector can be initialized randomly, and then updated iteratively during training. Generally, an autoencoder is a type of artificial neural network used to learn efficient data coding in an unsupervised manner (e.g., learns to copy its input to its output). The aim of an autoencoder is to learn a representation (encoding) for a set of data, typically for dimensionality reduction, by training the network to ignore signal "noise". Along with the reduction side, a reconstructing side is learned, where the autoencoder tries to generate from the reduced encoding a representation as close as possible to its original input, hence its name. Performing the copying task perfectly would simply duplicate the signal, and this is why autoencoders usually are restricted in ways that force them to reconstruct the input approximately, preserving only the most relevant aspects of the data in the copy.

As shown in FIG. 5, the inputs 512 and 514 are provided to a hidden layer 530 depicted as including nodes 532, 534, 536, and 538. The neural network 500 performs the processing via the hidden layer 530 of the nodes 532, 534, 536, and 538 to exhibit complex global behavior, determined by the connections between the processing elements and element parameters. Thus, the transition between layers 510 and 530 can be considered an encoder stage that takes the inputs 512 and 514 and transfers it to a deep neural network (within layer 530) to learn some smaller representation of the input (e.g., a resulting the latent representation).

The deep neural network can be a CNN, a non-CNN, a LSTM neural network, a fully connected neural network, or combination thereof. The inputs 512 and 514 can be IC ECG, body surface ECG, or IC ECG and body surface ECG. This encoding provides a dimensionality reduction of the inputs 512 and 514. Dimensionality reduction is a process of reducing the number of random variables (of the inputs 512 and 514) under consideration by obtaining a set of principal variables. For instance, dimensionality reduction can be a feature extraction that transforms data (e.g., the inputs 512 and 514) from a high-dimensional space (e.g., more than 10 dimensions) to a lower-dimensional space (e.g., 2-3 dimensions). The technical effects and benefits of dimensionality reduction include reducing time and storage space requirements for the data 410, improving visualization of the data 410, and improving parameter interpretation for ML. This data transformation can be linear or nonlinear. The operations of receiving (block 520) and encoding (block 525) can be considered a data preparation portion of the multi-step data manipulation by (the autoencoder of) the mapping engine 101.

At block 545 of the method 510, the neural network 500 decodes the latent representation. The decoding stage takes the encoder output (e.g., the resulting the latent representation) and attempts to reconstruct some form of the inputs 512 and 514 using another deep neural network. In this regard, the nodes 532, 534, 536, and 538 are combined to produce in the output layer 550 an output 552, as shown in block 560 of the method 510. That is, the output layer 590 reconstructs the inputs 512 and 514 on a reduced dimension but without the signal interferences, signal artifacts, and signal noise. Examples of the output 552 include cleaned biometric data (e.g., clean/denoised version of IC ECG data or the like). For instance, the denoised version of IC ECG could be free from one or more of the following: far field reduction, power line noise, contact noise, deflection noise, baseline wonders, respiration noise, and Fluro noise. The technical effects and benefits of the cleaned biometric data include enabling more accurate monitor, diagnosis, and treatment any number of various diseases.

For instance, target data for the output layer 550 includes target data type one ventricular activity (Y1) and includes target data type two input data after far field reduction (Y2). In accordance with an embodiment, the autoencoder of the mapping engine 101 can be a denoising autoencoder to find mapping functions (f, g) such that $f(X) = Y1$ and $g(X) = Y2$. Any combination of software and/or hardware (e.g., the local computing device 206 and the remote computing system 208, along with the apparatus 204) can individually or collectively store, execute, and implement the denoising autoencoder and functions thereof. The denoising autoencoder trains the autoencoder to reconstruct an input from a corrupted version of itself to force the hidden layer (e.g., the hidden layer 530 of FIG. 5) to discover more robust features (i.e., useful features that will constitute better higher level representations of the input) and prevent it from learning a particular identity (i.e., always returning to a same value). In this regard, the denoising autoencoder encodes the input (e.g., to preserve information about the input) and reverses the effect of a corruption process stochastically applied to the input of the autoencoder. According to one or more embodiments, IC ECG data compression can be performed using an autoencoder of the mapping engine 101. For instance, IC ECG data recorded during an electrophysiology procedure requires a significant amount of data storage and other resources to store and share this data for research purposes. The mapping engine 101 reduces resources needed for storage and sharing of the IC ECG data.

Returning to FIG. 3, at block 330, the mapping engine 101 initiates a procedure. The mapping engine can initiate a medical procedure for the patient 125. The medical procedure can be any procedure, treatment, and/or combination thereof as described herein. In an example the medical procedure can be a cardiac electrophysiology procedure for examining/mapping the heart 120 and treating arrhythmia types.

At block 335, the mapping engine 101 receives information. The mapping engine can receive information with respect to initiating a medical procedure for a patient (at block 330), such as patient set-up details, catheter information, area of interest, and other details relating to the medical procedure. The information can also include health demographics and biometric data of the patient 125. In some cases, the system 100 includes an eye tracking apparatus worn by the one or more users while initiating (and conducting) the medical procedure, which monitors and obtains the information.

At block 360, the mapping engine 101 predicts one or more workflows. The mapping engine 101 can predict workflow preferences for the physician 115 (e.g., the CAS, user, or the like) performing the medical procedure from the information by employing the model of the mapping engine 101. In some cases, the mapping engine 1010 predicts next events to be performed by the physician 115.

At block 365, the mapping engine 101 outputs image data (e.g., setup and settings particular to the physician 115). According to one or more embodiments, the mapping engine 101 can generate, using the workflow preferences (at block 360), image display options as the image data to the physician 115 for presentation by the system 100. Generally, the image display options are presented to the physician 115 (e.g., the CAS, user, or the like) via one or more displays 165 during the medical procedure. The image display options can a graphical control element, such as a toolbar, a tab, or an intelligent toolbar, with input/output element therein. The input/output elements can include buttons and/or icons. The image display options can also include one or more screens, one or more pop-up windows, one or more visualizations, one or more templates, one or more presentations, one or more menus, one or more maps, or one or more tools.

In an example, the mapping engine 101 can adjust (block 365) the GUI of the system 100 based upon relevant arrhythmia types, relevant physician preferences (block 360), and patient's demographics and potential other parameters. Further, the mapping engine 101 through the GUI (block 365) can support the workflow and can aid the physician 115 by opening and closing windows, setting transparencies, changing maps, etc., in a manner that is intuitive and preferred by the physician 115. The mapping engine 101 may also be used to provide the system 100 with an intelligent toolbar that provides buttons and options based upon the physician's personal preferences previously obtained.

According to one or more embodiments, the physician's personal preferences (obtained and identified at block 320) can be provided as the prediction. For example, during arrhythmia treatments, each time the physician 115 (e.g., CAS, user, or the like) would prepare to click on a button (e.g., "SWITCH TO BIPOLAR MAP", "POINT ACQUISITION", "FILTER SETUP", etc.), the mapping engine 101 can predict this action and highlight and/or click the button in advance. Further, when information is saved/fed to RNN AI transformers of the mapping engine 101, context detection can be applied with respect to the predictions. Note that RNN AI transformers can include deep learning models that adopt mechanisms of attention, differentially weighing significance of input data parts. In this regard, contextual events, such as "ABLATION STARTED", "ABLATION ENDED", "SIMULATION STARTED", "STIMULATION ENDED", changes in the cardiac cycle length, changes in the arrhythmia can be anticipated and predicted by the mapping engine 101. For instance, during the initiated medical procedure, the RNN AI transformers of the mapping engine follow button clicks of the buttons and adds items to an intelligent toolbar of the GUI. For example, the mapping engine 101 may learn that after a change in the cycle length, a probability that the physician will press on the "NEW MAP" button is high and can therefore highlight/present/add this button.

According to one or more embodiments, the mapping engine 101 can generate, within the image display options, input/output elements for the next events based on the probabilities for selection by the physician 115. The input/output elements can correspond with a predetermined number of next user events or a predetermined number of next hybrid events having a highest probability to occur or a probability to occur above a predetermined threshold.

Alternatively or in addition to tracking the physician 115 clicking on buttons during, for example, an arrhythmia treatment, eye tracking technology can track a gaze of the physician 115 onto the various windows, maps, screens, etc., of the GUI of the system 100. The eye tracking technology may be used to enable the system 100 to understand what is important on a particular cardiac map. Once the system 100 has observed and learned, the mapping engine 101 can automatically suggest/predict a next step and adjust the GUI accordingly. For example, if the physician 115 is concluding an ablation of a left PV, the system 100 can adjust a map viewer of the GUI to a view that is better optimized to a right PV. All predictions and subsequent user actions can by uploaded/stored within the system 100 for use by other users.

Note that the method 300 can end and/or repeat.

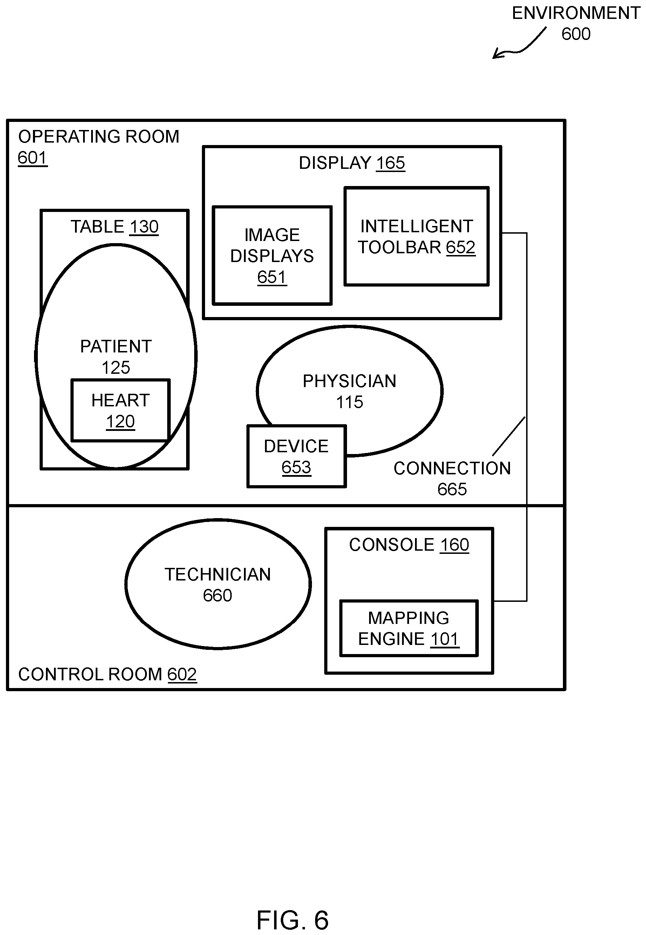

FIG. 6 illustrates a diagram of an environment 600 in which an exemplary system (e.g., the system 100 of FIG. 1) is utilized according to one or more embodiments. Note that some elements and items of FIG. 1 are shown and reused in FIG. 6 for ease of explanation and brevity.

The environment 600 illustrates an operating room 601 and a control room 602. The operating room 601 includes the physician 115 performing a medical procedure on the patient 125 on a table 130. The display 165 presents image displays 651 and an intelligent toolbar 652. The physician 115 can wear a device 653, such an eye tracking apparatus as described herein. The control room 602 includes a technician 660 who is operating the console 160 including the mapping engine 101 that has a connection 665 to the display 165. In some cases, the display 165 can be a simplified touch screen that contains the intelligent toolbar 652.

Generally, the environment 600 can include the CARTO®3 3D mapping system implemented through portions of the system 100 depicted therein. Such mapping systems incorporate advanced imaging technology that utilizes electromagnetic technology to create real-time three-dimensional (3D) maps (e.g., image displays 651) of a patient's cardiac structures. Such systems are designed to help electrophysiologists (e.g., the physician 115) navigate the heart 120 by generating an accurate 3D map, as well as pinpointing the exact location and orientation of catheters 110 in the heart 120 during diagnostic and therapeutic procedures for patients 125 suffering from heart rhythm conditions (cardiac arrhythmias). During a therapeutic catheter ablation procedure, the physician 115 inserts the catheter 110 of FIG. 1 through a small incision in the groin where it is then weaved up to the heart 120 through a blood vessel in the leg. Once it reaches the heart 120, RF energy is delivered to specific areas of the heart wall to produce a small lesion, or scar, to block faulty electrical impulses that can cause heart rhythm disorders.

According to one or more embodiments, the physician 115 may not have access to a keyboard, mouse, or other input devices of the console 160 as the operating room 601 and the control room 602 may be divided into a sterile side where medical procedures (e.g., a cardiac mapping and ablation procedures) are conducted and a semi-sterile side respectively. The technician 660 (e.g., a CAS or other user) has control of the system 100 via the console 160, while only predicted buttons of the intelligent toolbar 652 are offered to the physician 115. This is in part because the physician 115 has gloves, is holding the probe 105, is busy with the medical procedure, and cannot use the console 160. Note that a transparent window can located between the operating room 601 and the control room 602.

The image displays 651 that is generated by the system 100 helps the physician 115 steer the catheter 110 of FIG. 1 to areas in the heart 120 where RF energy needs to be administered. According to one or more advantages, technical effects, and/or benefits, the image displays 651 and the intelligent toolbar 652 of the environment 600 readily present predicted and/or preferred options in the GUI to the physician 115 and the technician 660 based upon a type of arrhythmia to be addressed, a patient's unique health demographics, and unique preferences.

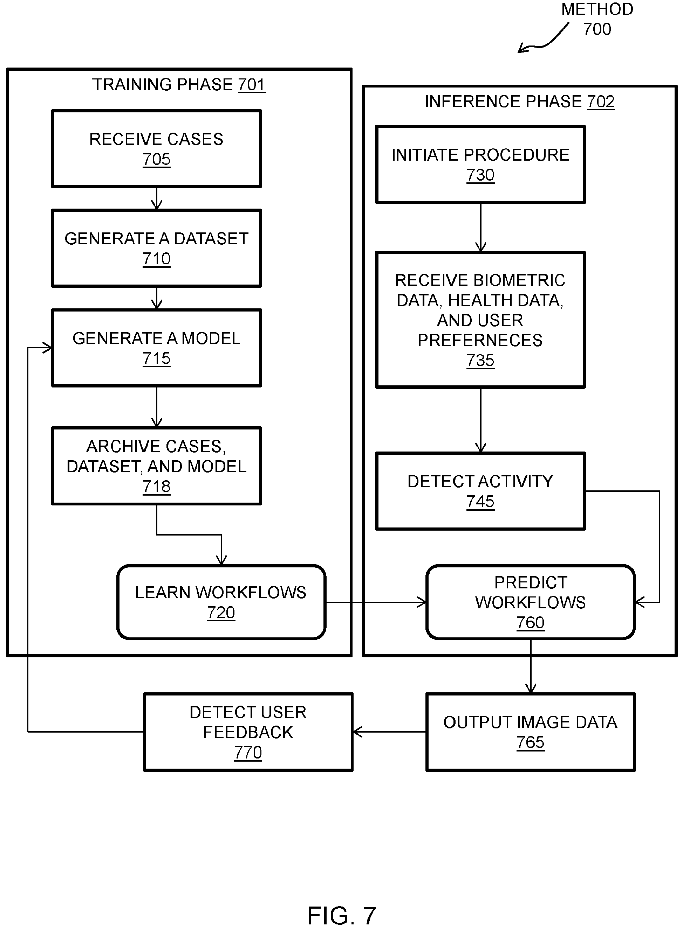

FIG. 7 illustrates a block diagram of a method 700 according to one or more embodiments. The method 700 is describes an example operation of the environment 600 with respect to the mapping engine 101. More particularly, the example operation of the method 700 provides a personalized GUI for the intelligent toolbar 652 and the console 160 based on a personalized workflow.

The method 700 includes a training phase 701 and an inference phase 702. The training phase 701 refers to the process of creating ML aspects of the mapping engine 101. The inference phase 702 refers to the process of applying (via AI) the ML aspects to make a prediction.

The training phase 701 of the process flow 700 begins at blocks 705, 710, and 715, where the mapping engine 101 receives cases, generates a dataset, and generates a model as described herein. Part of the operations of blocks 705, 710, and 715 can include personalized workflow and triggered case information archiving.

In conventional cardiac mapping and ablation system, all of the details of a cardiac ablation procedure are recorded on a hard drive provided with the conventional cardiac mapping and ablation system. For instance, the hard drive is connected to an external storage device that downloads the cardiac ablation procedure details to the external storage device during the course of the entire procedure, including the setup time and after the physician has completed the ablation. A drawback with this approach is that all details are being recorded regardless of their interest to physicians seeking to improve patient outcomes. For example, details relating to patient setup or removal of catheters at the conclusion of the cardiac ablation procedure may not be of interest to physicians seeking to improve patient outcomes. Another drawback with this approach is that because the download process is ongoing and involves a large amount of data, conducting the download process during progression of the cardiac ablation procedure can place undue burden on resources of the conventional cardiac mapping and ablation system resulting in delay during the cardiac ablation procedures.

At block 718 of the training phase 701, the system 100 archives cases, datasets, and models in a memory (e.g., the memory 162 of FIG. 1, cloud-based device, accessible through an external storage service, etc.), such as after a medical procedure. For example, archiving details of each completed cardiac mapping and ablation case (e.g., completed medical procedures) gives physicians and electrophysiology lab administrators the ability to review, analyze, and share cases and case data with the goal of helping to improve procedure efficiency, lab efficiency, and patient outcomes. For instance, cardiac ablation procedures can usually take three to six hours to complete, while complicated procedures may take longer. Often, it is difficult to predict how long a cardiac procedure will take to reach its conclusion, either at the start of the procedure or during the course of the procedure. Note that medical procedures, in general, include setup time during which the technician 600 (and other medical personnel, like nurses) ready the patient 125 while the physician 115 may not even be in the operating room 601. Likewise, after the physician 115 has completed the cardiac ablation procedure, time is spent by the technician 600 (and other medical personnel, like nurses) removing the catheter 110 of FIG. 1 from the patient 125.

At block 720 of the training phase 701, the mapping engine 101 learns one or more workflows. For instance, the mapping engine, through ML, the cardiac mapping system can learn workflow of the medical procedure based upon prior knowledge including (1) the particular preferences of different physicians in conducting cardiac mapping and ablations, (2) the unique characteristics of the patient's arrhythmia, and (3) the patient's personal health demographics. In this manner, the mapping engine 101 can determine whether setup is occurred during a course of the medical procedure and/or make decisions about whether to archive data during the setup step. For example, if the technician 600 (and other medical personnel, like nurses) are setting up the patient, this may not be of interest and the mapping engine 101 can determine not to archive data relating to the setup, thus reducing the amount of data stored in archive. That is, by knowing what step of the procedure is of interest, and which are not, the amount of data archived can be reduced.

As another example of block 720, the mapping engine 101 can accurately determine when a conclusion of the medical procedure is approaching so that the archival process may begin as the conclusion of the procedure approaches. In this manner, resources of the system 100 are free during most of the medical procedure, which has already occurred, and significant system resources are not spent on archiving after the conclusion of the procedure.

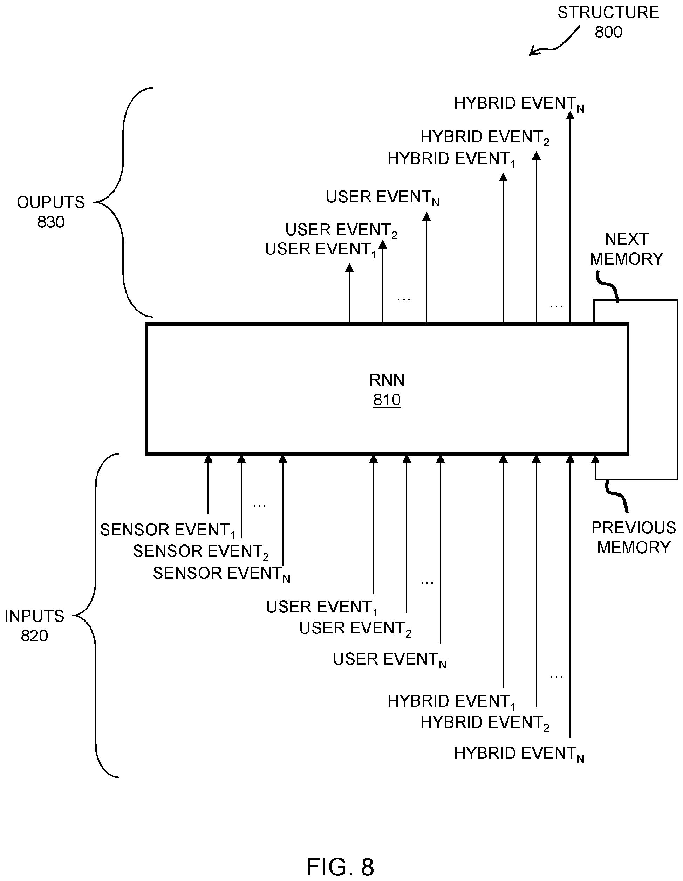

According to one or more embodiments, learning one or more workflows at block 720 can be performed by an RNN of the mapping engine 101. FIG. 8 illustrates a structure 800 of a RNN 810 according to one or more embodiments.

Generally, an RNN is a specific type of neural network, which is a specific type of ML. LSTM neural networks are subtype of RNN networks. One distinction from a neural network that learns to give an output for each input, RNNs learn to tell the next element in a sequence of infinite length. For example, during the learning phase, the RNN takes input-output pairs as follows:

- the → president
- president → is
- is → in
- in → the
- the → white
- white → house

The RNN has a memory and therefore, all previous inputs are also taken into consideration. To be more precise, the RNN takes input-output pairs as illustrated below:

- memory + the → president
- memory + president → is
- memory + is → in
- memory + in → the
- memory + the → white
- memory + white → house

Further, RNN networks remember the effect of the history in a sequence of infinite length. In other words, the memory of the RNN is affected by all previous elements in the sequence. If the RNN learns only the sentence above, every time it sees the word "the", it will suggest adding the word "president" or "house". However, in the context of auto-completion of sentences, the RNN may learn millions of sentences from newspapers, books, Wikipedia, journals etc. Once the RNN learns enough, it is able to predict the next word quite accurately.

Also, the RNN is able to suggest multiple "next moves", each one with a confidence level. For example, after the sentence "The announcement is made in the white", the RNN may suggest:

- House: 97%
- Shirt: 2%
- Bread: 1%

The mapping engine 101 can show a few predictions with the highest confidence level (e.g., the first 3) and/or above some confidence threshold (e.g., above 50%). Particularly, the mapping engine 101 can provide this autocomplete capability in the GUI, such as through the intelligent toolbar 652.

More particularly, as shown in the structure 800, various events (e.g., user events, sensor events, and hybrid events) serve as inputs 820 to the RNN 810. The inputs 820 can also include specific procedure type and a state of tachycardia.

Examples of user events include, but are not limited to, (1) the user clicked on the ACQUIRE button; (2) the user clicked on the NEW MAP button; (3) the user hides the ADDITIONAL MAP window; (4) the user moves the POINT LIST to the left; (5) the user changes the color of the REF catheter as "ORANGE"; etc.

Examples of sensor events include, but are not limited to, (1) ABLATION STARTED event detected; (2) PACING STARTED event detected; (3) a beats per minute (BPM) value between 100 and 149 detected; (4) a BPM value between 150 and 199 detected; (5) a BPM value between 200 to 249 detected; (6) a BPM increase at least by 25% in the last 60 seconds detected; (7) a BPM decrease at least by 25% in the last 60 seconds detected; (8) the eye tracker detects that the user (physician) is looking into the ADDITIONAL MAP window; (9) the eye tracker detects that the user is looking into the POINT LIST window; etc. Alternatively, some sensor events may be: (1) ABLATION IS ON; and, (2) PACING IS ON. It is important to mention that, unlike the "ABLATION STARTED" and "ABLATION ENDED" events that are triggered only once, the "ABLATION IS ON" event is triggered continuously all along the way during the ablation being turned on.

Examples of Hybrid events include, but are not limited to, events detected from a sensor. However, unlike sensor events where the physician has no control (e.g., blood pressure increase detected), hybrid events may be reproduced by the physician. Thus, hybrid events include, but are not limited to, (1) connection to the PENTARAY® catheter detected; (2) connection to the PICASSO catheter detected; etc. Hybrid events may be associated with a message or action, such as (1) "Connection of the PENTARAY® catheter detected → Do you want to connect a PENTARAY® catheter" or (2) "Connection of the PICASSO catheter detected → Do you want to connect a PICASSO catheter?"

Every user event, sensor event, and hybrid event can be represented by a binary input 820 to the RNN 810 ("1" when the event is detected, "0" at any other time). Every user event and hybrid event (but not sensor events) can be an output 830 to the RNN 810.

More particularly, as shown in the structure 800, outputs 830 of an RNN network are positive or negative real numbers of any amplitude. To obtain the probabilities of each output, the outputs 830 can be normalized via a softmax function. After the application of the softmax function, the sum of all outputs will always be equal to 100%. The softmax function gives the probability of every output as a number between 0% and 100%. The softmax function is defined as Equation 1:

$$Softmax(x_i) = \frac{exp(x_i)}{\sum_j exp(x_j)} \qquad\qquad \text{Equation 1}$$

where $x$ values are the outputs, $j$ is the index that iterates over every output, $i$ is the index of the output whose probability is calculated, and $exp(x_i)$ is the natural exponential function defined as $e$ (2.71828...) to the power of $x$.

Thus, during the training phase 701, all events are fed into the RNN 801. The RNN 801 learns the sequence of events. Further, the mapping engine 101 analyzes numerous cardiac mapping and ablation cases (from block 705) so that input-output pairs can be fed to the RNN 810 as the sequence of events. The learning may be performed world-wide, per hospital, per physician 115 (e.g., a physician on site, in a region, in a country, per equipment, etc.), and/or per operating room 601. If the learning is to be performed world-wide, the learning can be performed in the cloud or at the premises of the manufacturer of the system 100. If the learning is to be performed per hospital, per physician 115, and/or per operating room 601, the learning can be performed on a server located on premises (within the environment 600). A pre-trained network can be provided as a default RNN. The pre-trained network can be further trained with local cases. Note that providing a pre-trained network is an advantage of the system 100 herein. That is, it is likely a manufacturer of the system 100 can collect enough cases to train an RNN from zero. And, while a single hospital may not have enough cases to train an RNN from zero, the hospital can start with the default RNN pre-trained by the manufacturer of the system 100, and further customize this network with local cases.

The inference phase 702 of the process flow 700 includes at blocks 730 and 735, where the mapping engine 101 initiates a procedure (e.g., a medical procedure) and receives information (e.g., biometric data, health data, user preference data), as described herein. Further, the inference phase 702 of the process flow 700 includes at blocks 745 and 760, where the mapping engine 101 detects activity and predicts workflows.

Generally, in the inference phase 702, all events of the procedure (block 730) are fed to the RNN 810 continuously. That is, once the RNN 810 has learned enough in the training phase 701, it can be able to predict user or hybrid events. Thus, using information (block 735) and detected activity (745), the RNN 810 can be fed with all events that the physician 115 performs or the sensor detects. The RNN 810 then infers "possible next moves" of the physician 115.

"Possible next moves" may be populated in the intelligent toolbar 652. The RNN can also calculate a probability of each next event or move. The mapping engine 101 and/or the system 100 can display (e.g., output image data at block 765) a predetermined number of possible next events having the highest probability and/or the possible next events having a probability above a predetermined threshold. The mapping engine 101 and/or the system 100 can detect user feedback at block 770, which can be provided for further training, eye tracking related.

In an example, according to one or more embodiments, automation of the system 100 can utilize the device 653 of FIG. 6 for detecting events at block 745 of FIG. 7. That is, eye tracking of the device 653 is a sensor technology that makes it possible for the system 100 to know where the physician 115 is looking (e.g., tracks the gaze behavior of one or both eyes). Further, eye tracking of the device 653 can detect the presence, attention and focus of the physician 115, such as special interests or points of "fixation", which areas in which a gaze stops moving and lingers long enough for the physician 115 to process what is seen. In an example, eye tracking for the device 653 can be arranged for attachment to the eye, such as a special contact lens with an embedded mirror or magnetic field sensor, and the movement of the attachment is measured with the assumption that it does not slip significantly as the eye rotates. In another example, eye tracking for the device 653 may be incorporated into wearable glasses. Accordingly, eye tracking for the device 653 can be worn by the physician 115 and used to capture physician information-seeking behavior, thus providing an additional layer of information that was not previously available to conventional cardiac mapping and ablation systems. The physician 115 can be equipped with eye tracking eyewear for tracking a physician's gaze on the display 165. For example, in connection with the PV isolation procedure discussed herein, by providing eye tracking capability, the system 100 can track gaze behavior of the physician 115 conducting the medical procedure. For example, if the physician 115 is performing a cardiac mapping and decides upon a location for ablation, eye tracking can provide to the system 100 some insight into a physician's thought process in arriving at that target location for an ablation, such as whether the physician 115 considered other target locations within the heart 120 for the ablation, where those alternative targets are located, and why those locations considered and rejected before deciding upon this location for ablation.

As another example, pulmonary vein (PV) isolation is a procedure used to stop abnormal electrical signals in the heart 120 that cause heart rhythm problems. Under the medical procedure, multiple lesions can be created over the circumference of the pulmonary veins on both sides of the heart 120. When the physician 115 finishes ablation of the left pulmonary ventricle, the system 100 can adjust the map viewer to a view that is better optimized for the right pulmonary ventricle.

Under another example, the physician 115 may be looking at several lesions to determine which is incomplete. By utilizing the eye tracking technology, the system 100 can determine that multiple lesions are being considered by the physician 115 and on that basis may provide guidance to the physician 115 in the form of examples of lesions created by other physicians in the past for the physician's use in determining which lesion is incomplete. In this manner, the physician 115 may obtain the benefit of the thought process of other physicians confronted with similar problems in forming a complete lesion at this particular location within the heart 120.

According to one or more embodiments, the intelligent toolbar 652 of FIG. 6 (or in a similar GUI structure) provides a quick access to aspects of the system 100 with various buttons, icons, etc. In some case, the intelligent toolbar 652 can be empty. Further, each time the physician 115 clicks on a button (for example, "SWITCH TO BIPOLAR MAP", "POINT ACQUISITION", "FILTER SETUP", etc.), this information is fed to the RNN 810. Also contextual events, such as "ABLATION STARTED", "ABLATION ENDED", "STIMULATION STARTED", "STIMULATION ENDED", change in the cardiac cycle length, change in the arrhythmia, are fed to the RNN 810. Alternatively, this information can be collected retroactively. On runtime, the RNN 810 follows up the clicks of the buttons, and adds items to the intelligent toolbar 652 accordingly. For example, the RNN 810 may learn that after a change in the cycle length, the chances that the physician will press on the "New Map" button is high.

According to one or more embodiments, the intelligent toolbar 652 of FIG. 6 (or in a similar GUI structure) provides autocomplete and/or word completion feature in which the mapping engine 101 predicts what the physician 115 is typing. The physician 115 can typically indicate an acceptance of a suggestion. Autocomplete speeds up human-computer interactions when it correctly predicts the word a user intends to enter after only a few characters have been typed into a text input field.

According to one or more embodiments, the intelligent toolbar 652 of FIG. 6 (or in a similar GUI structure) can display buttons, messages, or suggestions.

For instance, the intelligent toolbar 652 of FIG. 6 (or in a similar GUI structure) can display the first N "possible next moves", such as a first three moves. And/or, the "possible next moves" can be populated based on a specific confidence threshold, such as 50% confidence. Note that the training phase 701 can provide the underlying values for calculating a probability, such that when an event happens that event receives a 1 and when an event does not happen that event receives a 0. Thus, in the inference phase 702, predictions can be generated based on training with confidence as a value for these predictions. Note that every new case is a new set of inputs to training.

Further, if the possible next move is a user event, then the user event can be displayed as a button in the intelligent toolbar 652 (e.g., the button is placed in a more visible place, with outlining or another marker or the like). For example, the "USER HAS HIDDEN THE ADDITIONAL MAP WINDOW" event can trigger displaying the "HIDE THE ADDITIONAL MAP WINDOW" button in the intelligent toolbar 652. The "USER CHANGED THE COLOR OF THE REF CATHETER AS ORANGE" can trigger displaying the "MAKE REF CATHETER ORANGE" button in the intelligent toolbar 652.

Furthermore, if the possible next move is a hybrid event, then a corresponding message may be displayed to the physician 115. For example, The "PENTARAYO CONNECTION DETECTED" event can trigger the "DO YOU WANT TO CONNECT A PENTARAY® CATHETER NOW?" message.

According to one or more example implementations, the environment 600 of FIG. 6 can include at least the following events:

E1 = eye tracker detected that the user is looking into the additional map window;

E2 = user pressed on the ACQUIRE button;

E3 = oxygen saturation (SpO2) of the patient is between 90% and 95%;

E4 = user has hidden the point list;

E5 = user changed the color of the ECG coming from the PENTARAY® catheter as green;

E6 = user pressed on the new map button;

E7 = physician started the ablation; and

E8 = blood pressure of the patient dropped below 100 mm Hg.

Accordingly, the RNN 810 of FIG. 8 learns to predict the next event, regardless of whether the event is a user event, a sensor event, or a hybrid event. For example, if the sequence is E1, E6, E3, E4, E4, E1, E6, the RNN 810 may predict "the next event will probably be E2". In some cases, the RNN 810 can make a distinction between a user event, a sensor event, and a hybrid event. For user events, if the RNN 810 thinks the next event is "the user will probably press on a specific button", as a design decision, the mapping engine 101 may be programmed to add this button to the intelligent toolbar 652, to make it easier for the user to find this button.

For sensor events, if the RNN thinks the next event is "the physician will probably start the ablation", a design decision may be made by the mapping engine 101 to do nothing with this prediction. In the event the mapping engine 101 senses that the physician 115 has started the ablation, this is information can be fed into the RNN 810 as an input 820, as it is part of the sequence of events. As part of the algorithm, the RNN 810 cannot ignore steps in the sequence of events that are performed. Yet, if the RNN 810 predicts that "the physician will probably start the ablation", no action is needed. In some cases, no buttons, messages, and/or questions need to be added to the intelligent toolbar 652. If the RNN 810 predicts the next event to be "the physician will probably start the ablation", in a naïve embodiment, this prediction can be ignored. However, in a more sophisticated embodiment, the mapping engine 101 can even prevent the RNN 810 from predicting this event in the first place. The RNN 810 can be built in a way that this event affects the sequence, as part of the input 820, but is not part of the output 830 (as illustrated in FIG. 8). In a naive embodiment, the RNN 810 may predict sensor events that the mapping engine 101 can ignore (do nothing with this prediction). In another embodiment, the RNN 810 can be built in a way that sensor events are not part of the output 830 pins at all.

For hybrid events, if the RNN 810 predicts that the next event is most probably "the eye tracker will detect that the physician will look into the additional map window", the mapping engine 101 can add to the intelligent toolbar 652 a button to maximize the additional map window. If the RNN 810 predicts that the next event is most probably "the physician will connect a PENTARAY® catheter", the mapping engine 101 can be programmed to issue a message stating "Please consider connecting a PENTARAY catheter". If the RNN 810 predicts that the next event is most probably "the blood pressure will drop below 100 mm Hg", the mapping engine 101 can be programmed to issue a message stating "Please monitor closely the blood pressure of the patient". Note that if events, like "the blood pressure dropped" and "the blood pressure will drop", are the same event, though one is stated in past tense as an input 820 and one stated in future tense as a prediction (output 830). In other words, the same event (E8 above) is stated in past tense when it is sensed by the RNN 810 an input 820, and in future tense when it is predicted or outputted 830 by the RNN 810.

Also, if the event E8 is defined as a sensor event, then when the RNN 810 predicts that "the next event will probably be E8", the mapping engine 101 can ignore this prediction (by definition... because this is what is done with sensor events). However, unlike sensor events, hybrid events are not ignored. The mapping engine 101 either displays a message or adds a specific button to the intelligent toolbar 652, or highlights a button, or asks a question, etc.

The flowchart and block diagrams in the Figures illustrate the architecture, functionality, and operation of possible

**EP 3 951 789 B1**

implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions. The invention is defined by the appended claims.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a RF transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof. The invention is defined by the appended claims.

The descriptions of the various embodiments herein have been presented for purposes of illustration, but are not intended to be exhaustive or limited to the embodiments disclosed. Many modifications and variations will be apparent to those of ordinary skill in the art as long as they stay within the scope of the appended claims, which define the invention.

**Claims**

1.  A machine learning system (100) comprising:

    a memory (162) configured to store processor executable program instructions of a mapping engine (101); and
    at least one processor (161) configured to execute the program instructions of the mapping engine to cause the system to:

    receive information (335) with respect to initiating a medical procedure for a patient (125), the information comprising health demographics and biometric data of the patient;
    predict (360) workflow preferences for one or more users performing the medical procedure from the information by employing a model (430) of the mapping engine trained using historical data (305) regarding past medical procedures on a plurality of patients, the historical data including:

    workflow preferences of a plurality of users performing the past medical procedures, and
    biometric data and/or health demographics of the plurality of patients; and

    generate (365), using the workflow preferences, image display options to the one or more users for presentation by the system;
    wherein predicting the workflow preferences comprises predicting next events to be performed by the one or more user conducting the medical procedure on the patient and predicting probabilities of the next events; and
    wherein generating the image display options comprises generating, within the image display options, input/output elements for the next events based on the probabilities for selection by the user.

**2.** The system of claim 1, wherein the system comprises an eye tracking apparatus (653) configured to be worn by a first user (115) of the one or more users while conducting the medical procedure, the eye tracking apparatus configured to monitor and obtain present workflow preferences of the first user.

**3.** The system of claim 1, wherein the at least one processor is further configured to execute the program instructions of the mapping engine to cause the system to:
initiate storage of the information relating to the medical procedure to a storage device in advance of a predicted time for completion of the medical procedure.

**4.** A machine learning method (300) comprising:

receiving (335), by a mapping engine (101) executed by at least one processor (161), information with respect to initiating a medical procedure for a patient (125), the information comprising health demographics and biometric data of the patient;
predicting (360), by the mapping engine, workflow preferences for one or more users performing the medical procedure from the information by employing a model (430) of the mapping engine trained using historical data (305) regarding past medical procedures on a plurality of patients, the historical data including:

workflow preferences of a plurality of users performing the past medical procedures, and
biometric data and/or health demographics of the plurality of patients; and

generating (365), by the mapping engine using the workflow preferences, image display options to the one or more users for presentation by the system;
wherein predicting the workflow preferences comprises predicting next events to be performed by the one or more user conducting the medical procedure on the patient and predicting probabilities of the next events; and
wherein generating the image display options comprises generating, within the image display options, input/output elements for the next events based on the probabilities for selection by the user.

**5.** The system of claim 1 or the method of claim 4, wherein the image display options are presented to the one or more users via one or more displays (165) during the medical procedure, at least one of the image display options comprises an intelligent toolbar (652).

**6.** The method of claim 4, wherein an eye tracking apparatus (653) monitors and obtains present workflow preferences of a first user (115) of the one or more, the first user wearing the eye tracking apparatus while conducting the medical procedure.

**7.** The method of claim 4, wherein the method comprises:
initiating storage of the information relating to the medical procedure to a storage device in advance of a predicted time for completion of the medical procedure.

**8.** The method of claim 4, wherein the biometric data comprises unique physiological characteristics of one or more arrhythmias of the patient.

**9.** The method of claim 4, wherein the model of the mapping engine comprises a trained deep learning architecture using a recurrent neural network (810).

**10.** The system of claim 1, wherein the biometric data comprises unique physiological characteristics of one or more arrhythmias of the patient.

**11.** The system of claim 1, wherein the model of the mapping engine comprises a trained deep learning architecture using a recurrent neural network (810).

**Patentansprüche**

**1.** Maschinenlernsystem (100), umfassend:

einen Speicher (162), der konfiguriert ist, um Programmanweisungen einer Mapping-Engine (101), die durch einen Prozessor abgearbeitet werden können, zu speichern; und
mindestens einen Prozessor (161), der konfiguriert ist, um die Programmanweisungen der Mapping-Engine abzuarbeiten, um das System zu veranlassen zum:

Empfangen von Informationen (335) in Bezug auf ein Einleiten eines medizinischen Eingriffs bei einem Patienten (125), die Informationen umfassend Gesundheitsdemografien und biometrische Daten des Patienten;
Vorhersagen (360) von Arbeitsablaufpräferenzen für einen oder mehrere Benutzer, die den medizinischen Eingriff durchführen, aus den Informationen durch Einsetzen eines Modells (430) der Mapping-Engine, das unter Verwendung von historischen Daten (305) über vergangene medizinische Eingriffe bei einer Vielzahl von Patienten trainiert wurde, wobei die historischen Daten einschließen:

Arbeitsablaufpräferenzen einer Vielzahl von Benutzern, die die vergangenen medizinischen Eingriffe durchgeführt haben, und
biometrische Daten und/oder Gesundheitsdemografien der Vielzahl von Patienten; und
Erzeugen (365), unter Verwendung der Arbeitsablaufpräferenzen, von Bildanzeigeoptionen für den einen oder die mehreren Benutzer für eine Präsentation durch das System;
wobei das Vorhersagen der Arbeitsablaufpräferenzen das Vorhersagen von nächsten Ereignissen, die durch den einen oder die mehreren Benutzer, die den medizinischen Eingriff bei dem Patienten ausführen, durchgeführt werden sollen, und das Vorhersagen von Wahrscheinlichkeiten der nächsten Ereignisse umfasst; und
wobei das Erzeugen der Bildanzeigeoptionen das Erzeugen, innerhalb der Bildanzeigeoptionen, von Eingabe-/Ausgabeelementen für die nächsten Ereignisse basierend auf den Wahrscheinlichkeiten für eine Auswahl durch den Benutzer umfasst.

2.   System nach Anspruch 1, wobei das System eine Blickverfolgungseinrichtung (653) umfasst, die konfiguriert ist, um von einem ersten Benutzer (115) des einen oder der mehreren Benutzer während des Ausführens des medizinischen Eingriffs getragen zu werden, wobei die Blickverfolgungseinrichtung konfiguriert ist, um aktuelle Arbeitsablaufpräferenzen des ersten Benutzers zu überwachen und zu erhalten.

3.   System nach Anspruch 1, wobei der mindestens eine Prozessor ferner konfiguriert ist, um die Programmanweisungen der Mapping-Engine abzuarbeiten, um das System zu veranlassen zum:
Einleiten der Speicherung der Informationen im Zusammenhang mit dem medizinischen Eingriff in einer Speicherungsvorrichtung vor einem voraussichtlichen Abschlusszeitpunkt des medizinischen Eingriffs.

4.   Maschinenlernverfahren (300), umfassend:

Empfangen (335), durch eine Mapping-Engine (101), durch mindestens einen Prozessor (161) abgearbeitet, von Informationen in Bezug auf das Einleiten eines medizinischen Eingriffs für einen Patienten (125), die Informationen umfassend Gesundheitsdemografien und biometrische Daten des Patienten;
Vorhersagen (360), durch die Mapping-Engine, von Arbeitsablaufpräferenzen für einen oder mehrere Benutzer, die den medizinischen Eingriff durchführen, aus den Informationen durch Einsetzen eines Modells (430) der Mapping-Engine, das unter Verwendung von historischen Daten (305) über vergangene medizinische Eingriffe bei einer Vielzahl von Patienten trainiert wurde, wobei die historischen Daten einschließen:
Arbeitsablaufpräferenzen einer Vielzahl von Benutzern, die die vergangenen medizinischen Eingriffe durchgeführt haben, und
biometrische Daten und/oder Gesundheitsdemografien der Vielzahl von Patienten; und
Erzeugen (365), durch die Mapping-Engine unter Verwendung der Arbeitsablaufpräferenzen, von Bildanzeigeoptionen für den einen oder die mehreren Benutzer für eine Präsentation durch das System;
wobei das Vorhersagen der Arbeitsablaufpräferenzen das Vorhersagen von nächsten Ereignissen, die durch den einen oder die mehreren Benutzer, die den medizinischen Eingriff bei dem Patienten ausführen, durchgeführt werden sollen, und das Vorhersagen von Wahrscheinlichkeiten der nächsten Ereignisse umfasst; und
wobei das Erzeugen der Bildanzeigeoptionen das Erzeugen, innerhalb der Bildanzeigeoptionen, von Eingabe-/Ausgabeelementen für die nächsten Ereignisse basierend auf den Wahrscheinlichkeiten für eine Auswahl durch den Benutzer umfasst.

5.   System nach Anspruch 1 oder Verfahren nach Anspruch 4, wobei die Bildanzeigeoptionen dem einen oder den mehreren Benutzern während des medizinischen Eingriffs über ein oder mehrere Displays (165) präsentiert werden, wobei mindestens eine der Bildanzeigeoptionen eine intelligente Symbolleiste (652) umfasst.

6. Verfahren nach Anspruch 4, wobei eine Blickverfolgungseinrichtung (653) aktuelle Arbeitsablaufpräferenzen eines ersten Benutzers (115) des einen oder der mehreren überwacht und erhält, wobei der erste Benutzer die Blickverfolgungseinrichtung während des Durchführens des medizinischen Eingriffs trägt.

7. Verfahren nach Anspruch 4, wobei das Verfahren umfasst:
Einleiten der Speicherung der Informationen im Zusammenhang mit dem medizinischen Eingriff in einer Speicherungsvorrichtung vor einem voraussichtlichen Abschlusszeitpunkt des medizinischen Eingriffs.

8. Verfahren nach Anspruch 4, wobei die biometrischen Daten einzigartige physiologische Merkmale einer oder mehrerer Arrhythmien des Patienten umfassen.

9. Verfahren nach Anspruch 4, wobei das Modell der Mapping-Engine eine trainierte Deep-Learning-Architektur unter Verwendung eines rekurrenten neuronalen Netzes (810) umfasst.

10. System nach Anspruch 1, wobei die biometrischen Daten einzigartige physiologische Merkmale einer oder mehrerer Arrhythmien des Patienten umfassen.

11. System nach Anspruch 1, wobei das Modell der Mapping-Engine eine trainierte Deep-Learning-Architektur unter Verwendung eines rekurrenten neuronalen Netzes (810) umfasst.


**Revendications**

1. Système d'apprentissage automatique (100) comprenant :

   une mémoire (162) configurée pour stocker des instructions de programme exécutables par processeur d'un moteur de mappage (101) ; et
   au moins un processeur (161) configuré pour exécuter les instructions de programme du moteur de mappage pour amener le système à :

      recevoir des informations (335) par rapport à l'initiation d'une procédure médicale pour un patient (125), les informations comprenant des données démographiques de santé et des données biométriques du patient ;
      prédire (360) des préférences de flux de travail pour un ou plusieurs utilisateurs mettant en œuvre la procédure médicale à partir des informations en faisant appel à un modèle (430) du moteur de mappage entraîné à l'aide de données historiques (305) concernant des procédures médicales passées sur une pluralité de patients, les données historiques comportant :

         des préférences de flux de travail d'une pluralité d'utilisateurs mettant en œuvre les procédures médicales passées, et
         des données biométriques et/ou des données démographiques de santé de la pluralité de patients ; et
         générer (365), à l'aide des préférences de flux de travail, des options d'affichage d'image à l'utilisateur ou aux utilisateurs pour présentation par le système ;
         dans lequel la prédiction des préférences de flux de travail comprend la prédiction d'événements suivants à mettre en oeuvre par le ou les utilisateurs réalisant la procédure médicale sur le patient et la prédiction de probabilités des événements suivants ; et
         dans lequel la génération des options d'affichage d'image comprend la génération, au sein des options d'affichage d'image, d'éléments d'entrée/sortie pour les événements suivants en fonction des probabilités pour sélection par l'utilisateur.

2. Système selon la revendication 1, dans lequel le système comprend un appareil de suivi oculaire (653) conçu pour être porté par un premier utilisateur (115) parmi le ou les utilisateurs tout en réalisant la procédure médicale, l'appareil de suivi oculaire étant configuré pour surveiller et obtenir des préférences de flux de travail actuelles du premier utilisateur.

3. Système selon la revendication 1, dans lequel l'au moins un processeur est configuré en outre pour exécuter les instructions de programme du moteur de mappage pour amener le système à :

initier un stockage des informations se rapportant à la procédure médicale vers un dispositif de stockage avant un moment prédit pour l'achèvement de la procédure médicale.

4. Procédé d'apprentissage automatique (300) comprenant :

la réception (335), par un moteur de mappage (101) exécuté par au moins un processeur (161), d'informations par rapport à l'initiation d'une procédure médicale pour un patient (125), les informations comprenant des données démographiques de santé et des données biométriques du patient ;
la prédiction (360), par le moteur de mappage, de préférences de flux de travail pour un ou plusieurs utilisateurs mettant en œuvre la procédure médicale à partir des informations en faisant appel à un modèle (430) du moteur de mappage entraîné à l'aide de données historiques (305) concernant des procédures médicales passées sur une pluralité de patients, les données historiques comportant :

des préférences de flux de travail d'une pluralité d'utilisateurs mettant en œuvre les procédures médicales passées, et
des données biométriques et/ou des données démographiques de santé de la pluralité de patients ; et
la génération (365), par le moteur de mappage à l'aide des préférences de flux de travail, d'options d'affichage d'image à l'utilisateur ou aux utilisateurs pour présentation par le système ;
dans lequel la prédiction des préférences de flux de travail comprend la prédiction d'événements suivants à mettre en œuvre par le ou les utilisateurs réalisant la procédure médicale sur le patient et la prédiction de probabilités des événements suivants ; et
dans lequel la génération des options d'affichage d'image comprend la génération, au sein des options d'affichage d'image, d'éléments d'entrée/sortie pour les événements suivants en fonction des probabilités pour sélection par l'utilisateur.

5. Système selon la revendication 1 ou procédé selon la revendication 4, dans lequel les options d'affichage d'image sont présentées à l'utilisateur ou aux utilisateurs par l'intermédiaire d'un ou plusieurs affichages (165) pendant la procédure médicale, au moins l'une des options d'affichage d'image comprend une barre d'outils intelligente (652).

6. Procédé selon la revendication 4, dans lequel un appareil de suivi oculaire (653) surveille et obtient des préférences de flux de travail actuelles d'un premier utilisateur (115) du ou des premiers utilisateurs portant l'appareil de suivi oculaire tout en réalisant la procédure médicale.

7. Procédé selon la revendication 4, dans lequel le procédé comprend :
l'initiation de stockage des informations se rapportant à la procédure médicale vers un dispositif de stockage avant un moment prédit pour l'achèvement de la procédure médicale.

8. Procédé selon la revendication 4, dans lequel les données biométriques comprennent des caractéristiques physiologiques uniques d'une ou plusieurs arythmies du patient.

9. Procédé selon la revendication 4, dans lequel le modèle du moteur de mappage comprend une architecture d'apprentissage profond entraînée à l'aide d'un réseau neuronal récurrent (810).

10. Système selon la revendication 1, dans lequel les données biométriques comprennent des caractéristiques physiologiques uniques d'une ou plusieurs arythmies du patient.

11. Système selon la revendication 1, dans lequel le modèle du moteur de mappage comprend une architecture d'apprentissage profond entraînée à l'aide d'un réseau neuronal récurrent (810).

FIG. 1

SYSTEM
200

PATIENT 202

APPARATUS 204

| BIOMETRIC SENSOR 221 | PROCESSOR 222 |

| USER INPUT SENSOR 223 | MEMORY 224 |

| TRANSCEIVER 225 | MAPPING ENGINE 101 |

NETWORK 210

REMOTE COMPUTING SYSTEM 208

MAPPING ENGINE 101

LOCAL COMPUTING DEVICE 206

MAPPING ENGINE 101

NETWORK 211

FIG. 2

METHOD
300

INITIATE PROCEDURE
330

RECEIVE CASES
305

RECEIVE
INFORMATION
335

GENERATE A DATASET
310

GENERATE A MODEL
315

LEARN WORKFLOWS
320

PREDICT
WORKFLOWS
360

OUTPUT IMAGE DATA
365

FIG. 3

FIG. 4

SYSTEM
500

514          530          550

512

514

532

534

536

538

552

METHOD
501

| RECEIVING 520 | ENCODING 525 | DECODING 545 | OUTPUTTING 580 |

FIG. 5

ENVIRONMENT
600

OPERATING ROOM
601

DISPLAY 165

TABLE 130

IMAGE
DISPLAYS
651

INTELLIGENT
TOOLBAR 652

PATIENT
125

HEART
120

PHYSICIAN
115

DEVICE
653

CONNECTION
665

TECHNICIAN
660

CONSOLE 160

MAPPING
ENGINE 101

CONTROL ROOM 602

FIG. 6

METHOD
700

**TRAINING PHASE 701**

RECEIVE CASES
705

GENERATE A DATASET
710

GENERATE A MODEL
715

ARCHIVE CASES,
DATASET, AND MODEL
718

LEARN WORKFLOWS
720

**INFERENCE PHASE 702**

INITIATE PROCEDURE
730

RECEIVE BIOMETRIC
DATA, HEALTH DATA,
AND USER
PREFERNECES
735

DETECT ACTIVITY
745

PREDICT
WORKFLOWS
760

DETECT USER
FEEDBACK
770

OUTPUT IMAGE DATA
765

FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011201900 A1
- WO 2018134143 A1